# FASCICULE DE BREVET EUROPEEN

(11) **EP 2 566 445 B1**
(45) Date de publication et mention de la délivrance du brevet: **25.02.2015**
(21) Numéro de dépôt: 11730359.4
(22) Date de dépôt: 11.04.2011
(51) Int. Cl.: A61K 8/81, A61K 47/32, A61Q 1/00, A61Q 5/00, A61Q 17/04, A61Q 19/00, C08K 5/06, C08K 5/10, C08L 33/00

(54) **NOUVEAU LATEX INVERSE AUTO-INVERSIBLE, SON UTILISATION COMME AGENT ÉPAISSISSANT DANS UNE COMPOSITION COSMÉTIQUE**
NEUER SELBSTINVERTIERBARER INVERSER LATEX UND SEINE VERWENDUNG ALS VERDICKUNGSMITTEL IN EINER KOSMETISCHEN ZUSAMMENSETZUNG
NOVEL SELF-REVERSIBLE REVERSE LATEX, AND USE THEREOF AS A THICKENING AGENT IN A COSMETIC COMPOSITION

(30) Priorité: 06.05.2010 FR 1053534
(43) Date de publication de la demande: 13.03.2013
(73) Titulaire: Societe D'Exploitation De Produits Pour Les Industries Chimiques Seppic, 75007 Paris (FR)
(72) Inventeur: BRAUN, Olivier, F-81100 Castres (FR); MALLO, Paul, F-78290 Croissy-sur-Seine (FR)
(74) Mandataire: Conan, Philippe Claude
(86) Numéro de dépôt international: PCT/FR2011/050816
(87) Numéro de publication internationale: WO 2011/138533

(56) Documents cités:
- EP-A1- 0 987 017
- EP-A1- 1 623 697

## Description

La présente demande concerne des latex inverse eau dans huile auto-inversibles, leur procédé de préparation et leur application en tant qu'épaississant et/ou émulsionnant de produits de soins de la peau, du cuir chevelu et des cheveux ou pour la fabrication de préparations cosmétiques, dermopharmaceutiques ou pharmaceutiques.

Les latex inverses à base de polyélectrolytes dont l'acide 2-méthyl 2-[(1-oxo 2-propènyl) amino]1-propanesulfonique (dénommé aussi acide 2-acrylamido 2-méthyl propanesulfonique, ATBS ou AMPS) partiellement ou totalement salifié, ainsi que leur utilisation en cosmétique et/ou pharmacie ont fait l'objet de nombreuses demandes de brevet. Cependant, la présence de quantités importantes d'eau et d'huile dans ces latex inverses représente un inconvénient non négligeable en termes de volume, de coût et parfois de risques accrus et/ou d'effets toxiques.

Des solutions ont donc été développées pour augmenter la concentration en polyélectrolytes dans les latex inverses finaux par exemple en soumettant le milieu réactionnel, en fin de polymérisation, à une étape de distillation sous vide pour enlever une partie plus ou moins importante d'eau et d'huile. Cette distillation est cependant délicate à mettre en oeuvre car elle induit souvent une déstabilisation du latex inverse qu'il faut contrer par l'addition préalable d'agents stabilisants.

Les demandes de brevet européen EP 0 161 038 et EP 0 126 528 ainsi que la demande de brevet britannique GB 1 482 515 divulguent une telle utilisation de polymères stabilisants.

L'inconvénient de ces polymères stabilisants est qu'ils contiennent des alcools ou des glycols pouvant induire des problèmes environnementaux. De plus il se produit parfois une prise en masse du milieu réactionnel lors de l'étape de distillation, sans que ce phénomène n'ait jamais vraiment été expliqué, mais dont la conséquence certaine est la destruction du lot de latex inverse en cours de fabrication et un nettoyage pénible et coûteux du réacteur utilisé. Enfin, même quand la distillation se déroule correctement, les latex inverses obtenus s'inversent souvent difficilement lors de leur mise en oeuvre dans une phase aqueuse. Ils présentent aussi une viscosité élevée et parfois en leur sein des micro-gels. Ces inconvénients interdisent donc leur utilisation dans la fabrication de formulations cosmétiques. Pour pallier à ces inconvénients, les inventeurs ont développé un latex inverse divulgué dans la demande de brevet français publiée sous le numéro FR 2 879 607 comprenant de 50% à 80% massique d'un polyélectrolyte comportant de 0,01% à 10% molaire d'au moins une unité monomérique dérivée du composé de formule (A) :

C(R₁)(R₃)=C(R₂)-C(=O)-O-(CH₂-CH₂-O)ₙ-R₄ (A)

dans laquelle les radicaux R₁, R₂ et R₃, identiques ou différents représentent indépendamment les uns des autres un atome d'hydrogène ou un radical alkyle linéaire ou ramifié comportant de 1 à 4 atomes de carbone, le radical R4 représente un radical aliphatique linéaire ou ramifié, saturé ou insaturé, comportant de 6 à 30 atomes de carbone et n représente un nombre compris entre 1 et 50.

Cependant, lorsque l'on met en oeuvre ce latex inverse pour préparer une formulation épaissie, sa vitesse d'inversion dans la phase aqueuse, c'est-à-dire le temps nécessaire pour obtenir le développement maximal de la viscosité, reste assez faible ce qui signifie pour l'utilisateur une perte de temps qui est dommageable en phase industrielle. En effet il est bien connu que le temps d'inversion des latex inverses augmente considérablement en fonction de l'échelle d'utilisation. Par ailleurs la stabilité dans le temps des latex inverses décrits dans FR 2 879 607 n'est pas totalement satisfaisante. On observe en effet un phénomène de synérèse avec apparition d'huile en surface et d'un culot à base de polymère assez rapide pendant le stockage.

Les inventeurs ont donc cherché à développer des latex inverses qui ne présentent pas les inconvénients précités.

Selon un premier aspect, l'invention a pour objet une composition sous forme d'un latex inverse auto-inversible comprenant pour 100% de sa masse :
**a) -** De 50% massique à 70% massique d'un polyélectrolyte anionique (P) réticulé, obtenu par polymérisation :
   - D'au moins un monomère neutre de formule (I) : 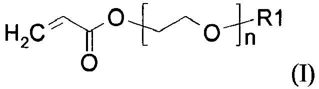 dans laquelle le radical R1 représente un radical aliphatique linéaire ou ramifié, comportant de 8 à 20 atomes de carbone et n représente un nombre supérieur ou égal à un et inférieur ou égal à trente ;
   - D'au moins un monomère neutre choisi parmi l'acrylamide, le N,N-diméthyl acrylamide, le N-[2-hydroxy-1,1-bis(hydroxyméthyl)éthyl] propènamide [ou tris(hydroxyméthyl) acrylamidométhane ou N-[tris(hydroxyméthyl) méthyl] acrylamide dénommé aussi THAM] ou l'acrylate de (2-hydroxy éthyle) ; et
   - D'au moins un monomère comportant une fonction acide fort et/ou d'au moins un monomère comportant une fonction acide faible ;
**b) -** De 4% massique à 10% massique d'un système émulsionnant (S₁) de type eau dans huile (E/H) ;
**c) -** De 1% massique à 10% massique d'un système émulsionnant (S₂) de type huile dans eau (H/E) comprenant une proportion massique non nulle d'une composition tensioactive (C), ladite composition tensioactive (C) comprenant pour 100% molaire :
   **1) -** Une proportion supérieure ou égale à 10% molaire et inférieure ou égale à 50% molaire d'une composition (C_{II}) comprenant pour 100% molaire :
      **α) -** De 60% molaire à 100% molaire d'un composé de formule (II) : 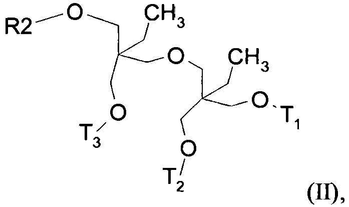 dans laquelle :
         - R2 représente un radical alkyle linéaire ou ramifié, comportant de 12 atomes de carbone,
         - T₁, représente un atome d'hydrogène ou un radical (-CH₂-CH₂-O-)ₘ₁-H dans lequel m1 est un nombre entier supérieur ou égal à zéro et inférieur ou égal à dix,
         - T₂ identique ou différent de T₁, représente un atome d'hydrogène ou un radical (-CH₂-CH₂-O-)ₘ₂-H dans lequel m2 est un nombre entier supérieur ou égal à zéro et inférieur ou égal à dix, et
         - T₃ identique ou différent de T₁ et de T₂, représente un atome d'hydrogène ou un radical (-CH₂-CH₂-O-)ₘ₃-H dans lequel m3 est un nombre entier supérieur ou égal à zéro et inférieur ou égal à dix,
         - étant entendu que la somme m1 + m2 + m3 est supérieure à 0 et inférieure ou égale à dix ; **β**) - Optionnellement jusqu'à 40% molaire d'un composé de formule (II') : 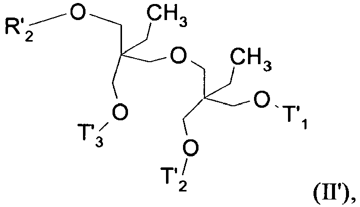
         dans laquelle :
         - R'₂ représente un radical alkyle linéaire ou ramifié, comportant 14 atomes de carbone,
         - T'₁, représente un atome d'hydrogène ou un radical (-CH₂-CH₂-O-)ₘ₁-H dans lequel m1 est un nombre entier supérieur ou égal à zéro et inférieur ou égal à dix,
         - T'₂ identique ou différent de T'₁, représente un atome d'hydrogène ou un radical (-CH₂-CH₂-O-)ₘ₂-H dans lequel m2 est un nombre entier supérieur ou égal à zéro et inférieur ou égal à dix, et
         - T'₃ identique ou différent de T'₁ et de T'₂, représente un atome d'hydrogène ou un radical (-CH₂-CH₂-O-)ₘ₃-H dans lequel m3 est un nombre entier supérieur ou égal à zéro et inférieur ou égal à dix,
         - étant entendu, que la somme m1 + m2 + m3 est supérieure à 0 et inférieure ou égale à dix ; et γ) - Optionnellement jusqu'à 10% molaire d'un composé de formule (II") : 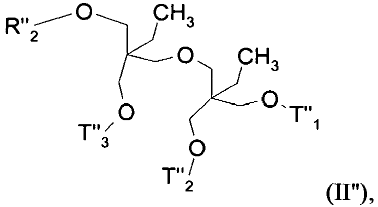
         dans laquelle :
         - R"₂ représente un radical alkyle linéaire ou ramifié, comportant 16 atomes de carbone,
         - T"₁, représente un atome d'hydrogène ou un radical (-CH₂-CH₂-O-)ₘ₁-H dans lequel m1 est un nombre entier supérieur ou égal à zéro et inférieur ou égal à dix,
         - T"₂ identique ou différent de T"₁, représente un atome d'hydrogène ou un radical (-CH₂-CH₂-O-)ₘ₂-H dans lequel m2 est un nombre entier supérieur ou égal à zéro et inférieur ou égal à dix, et
         - T"₃ identique ou différent de T"₁ et de T"₂, représente un atome d'hydrogène ou un radical (-CH₂-CH₂-O-)ₘ₃-H dans lequel m3 est un nombre entier supérieur ou égal à zéro et inférieur ou égal à dix,
         - étant entendu que la somme m1 + m2 + m3 est supérieure à 0 et inférieure ou égale à dix ;
   **2) -** Une proportion supérieure ou égale à 50% molaire et inférieure ou égale à 90% molaire d'une composition (C_{III}) comprenant pour 100% molaire :
      **α) -** De 60% molaire à 100% molaire d'un composé de formule (III) : 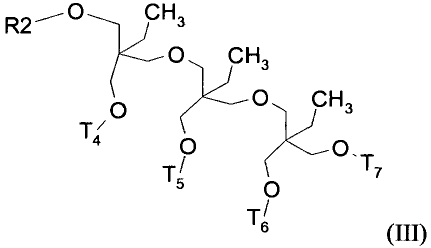 ou de son isomère de formule (IV) : 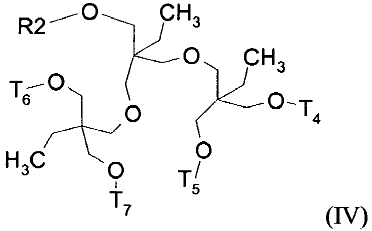 ou du mélange de ces deux isomères,
         formules (III) et (IV) dans lesquelles :
         - R2 représente un radical alkyle linéaire ou ramifié, comportant 12 atomes de carbone,
         - T₄, représente un atome d'hydrogène ou un radical (-CH₂-CH₂-O-)ₘ₄-H dans lequel m4 est un nombre entier supérieur ou égal à zéro et inférieur ou égal à dix,
         - T₅ identique ou différent de T₄, représente un atome d'hydrogène ou un radical (-CH₂-CH₂-O-)ₘ₅-H dans lequel m5 est un nombre entier supérieur ou égal à zéro et inférieur ou égal à dix,
         - T₆ identique ou différent de T₄ et de T₅ représente un atome d'hydrogène ou un radical (-CH₂-CH₂-O-)ₘ₆-H dans lequel m6 est un nombre entier supérieur ou égal à zéro et inférieur ou égal à dix,
         - T₇ identique ou différent de T₄ de T₅ et de T₆, représente un atome d'hydrogène ou un radical (-CH₂-CH₂-O-)ₘ₇-H dans lequel m7 est un nombre entier supérieur ou égal à zéro et inférieur ou égal à dix étant entendu que la somme m4 + m5 + m6 + m7 est supérieure à 0 et inférieure ou égale à dix ;
      **β) -** Optionnellement jusqu'à 40% molaire composé de formule (III') : 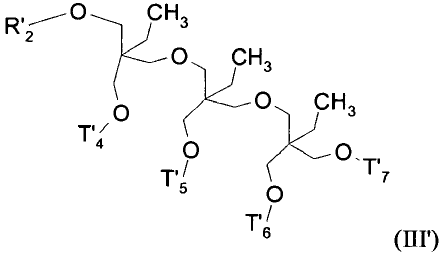 ou de son isomère de formule (IV') : 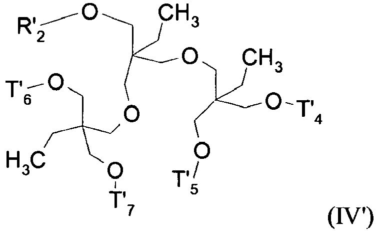 ou du mélange de ces deux isomères,
         formules (III') et (IV') dans lesquelles :
         - R'₂ représente un radical alkyle linéaire ou ramifié, comportant 14 atomes de carbone,
         - T'₄, représente un atome d'hydrogène ou un radical (-CH₂-CH₂-O-)ₘ₄-H dans lequel m4 est un nombre entier supérieur ou égal à zéro et inférieur ou égal à dix,
         - T'₅ identique ou différent de T'₄, représente un atome d'hydrogène ou un radical (-CH₂-CH₂-O-)ₘ₅-H dans lequel m5 est un nombre entier supérieur ou égal à zéro et inférieur ou égal à dix,
         - T'₆ identique ou différent de T'₄ et de T'₅, représente un atome d'hydrogène ou un radical (-CH₂-CH₂-O-)ₘ₆-H dans lequel m6 est un nombre entier supérieur ou égal à zéro et inférieur ou égal à dix, et
         - T'₇ identique ou différent de T'₄, de T'₅ et de T'₆, représente un atome d'hydrogène ou un radical (-CH₂-CH₂-O-)ₘ₇-H dans lequel m7 est un nombre entier supérieur ou égal à zéro et inférieur ou égal à dix,
         - étant entendu que la somme m4 + m5 + m6 + m7 est supérieure à 0 et inférieure ou égale à dix ; et
      **γ) -** Optionnellement jusqu'à 10% molaire d'un composé de formule (III") : 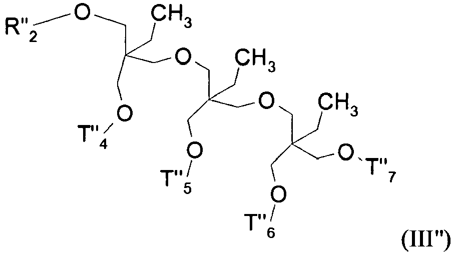 ou de son isomère de formule (IV") : 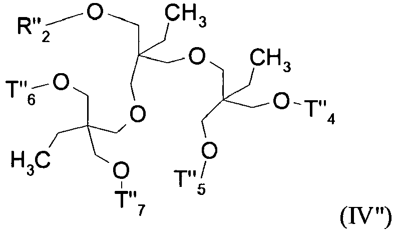 ou du mélange de ces deux isomères,
         formules (III") et (IV") dans lesquelles :
         - R"₂ représente un radical alkyle linéaire ou ramifié, comportant 16 atomes de carbone,
         - T"₄, représente un atome d'hydrogène ou un radical (-CH₂-CH₂-O-)ₘ₄-H dans lequel m4 est un nombre entier supérieur ou égal à zéro et inférieur ou égal à dix,
         - T"₅ identique ou différent de T"₄, représente un atome d'hydrogène ou un radical (-CH₂-CH₂-O-)ₘ₅-H dans lequel m5 est un nombre entier supérieur ou égal à zéro et inférieur ou égal à dix,
         - T"₆ identique ou différent de T"₄ et de T"₅ représente un atome d'hydrogène ou un radical (-CH₂-CH₂-O-)ₘ₆-H dans lequel m6 est un nombre entier supérieur ou égal à zéro et inférieur ou égal à dix, et
         - T"₇ identique ou différent de T"₄, de T"₅ et de T"₆, représente un atome d'hydrogène ou un radical (-CH₂-CH₂-O-)ₘ₇-H dans lequel m7 est un nombre entier supérieur ou égal à zéro et inférieur ou égal à dix,
         - étant entendu que la somme m4 + m5 + m6 + m7 est supérieure à 0 et inférieure ou égale à dix ;
      **d) -** De 15% massique à 45% massique d'au moins une huile, et
      **e) -** De 0% massique à 5% massique d'eau.

Par radical aliphatique linéaire ou ramifié, saturé ou insaturé, comportant de 6 à 20 atomes de carbone, on désigne pour le radical R1 dans la formule (I) telle que définie ci-dessus, plus particulièrement les radicaux linéaires tels que, par exemple, les radicaux, hexyle, octyle, nonyle, décyle, undécyle, dodécyle, tétradécyle, hexadécyle, octadécyle ou eicosyle

Selon un premier aspect particulier, dans la formule (I) telle que définie ci-dessus, le radical R1 représente un radical lauryle ou un radical stéaryle.

Selon un autre aspect particulier, dans la formule (I) telle que définie ci-dessus, n est supérieur ou égal à deux et inférieur ou égal à vingt.

Selon un autre aspect particulier, dans le polyélectrolyte P de la composition objet de la présente invention, la fonction acide fort des monomères en comportant est notamment la fonction acide sulfonique. Lesdits monomères sont par exemple l'acide styrènesulfonique partiellement ou totalement salifié ou, l'acide 2-méthyl 2-[(1-oxo 2-propènyl) amino] 1-propanesulfonique (dénommé aussi acide 2-acrylamido 2-méthyl propanesulfonique) partiellement ou totalement salifiée.

Selon un autre aspect particulier, dans le polyélectrolyte P compris dans la composition objet de la présente invention, la fonction acide faible des monomères en comportant est notamment la fonction acide carboxylique partiellement salifiée. Lesdits monomères sont par exemple l'acide acrylique, l'acide méthacrylique, l'acide itaconique, l'acide maléique ou l'acide 3-méthyl 3-[(1-oxo 2-propènyl) amino] butanoïque partiellement ou totalement salifié. Il s'agit plus particulièrement de l'acide acrylique ou de l'acide méthacrylique partiellement salifié.

Pour les monomères à fonction acide fort ou à fonction acide faible, le terme salifié indique qu'il s'agit de sels de métaux alcalins tels que les sels de sodium ou de potassium, les sels de bases azotées comme, ou le sel d'ammonium.

L'invention a plus particulièrement pour objet, une composition telle que définie précédemment, pour laquelle dans le polyélectrolyte (P), les unités monomériques comportant une fonction acide fort sont issues de l'acide 2-méthyl 2-[(1-oxo 2-propènyl) amino] 1-propanesulfonique partiellement ou totalement salifié en sel de sodium, en sel de potassium, ou en sel d'ammonium et les unités monomériques comportant une fonction acide faible sont issues de l'acide acrylique ou de l'acide méthacrylique, partiellement salifié en sel de sodium, en sel de potassium, ou en sel d'ammonium.

L'invention a aussi plus particulièrement pour objet une composition telle que définie précédemment, pour laquelle le polyélectrolyte P comprend, en pourcentage molaire, de 0,5% à 10% d'une unité monomérique issue du monomère de formule (I) telle que définie précédemment.

Selon un autre aspect particulier de la présente invention, ladite composition tensioactive (C) telle que définie précédemment, comprend en outre :
**3) -** Jusqu'à 5% molaire d'une composition (C_{V}) comprenant pour 100% molaire
   **α) -** De 60% molaire à 100% molaire d'un composé de formule (V) : 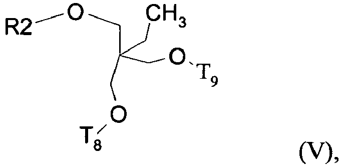 dans laquelle :
      - R2 représente un radical alkyle linéaire ou ramifié, comportant de 12 atomes de carbone,
      - T₈, représente un atome d'hydrogène ou un radical (-CH₂-CH₂-O-)ₘ₈-H dans lequel m8 est un nombre entier supérieur ou égal à zéro et inférieur ou égal à dix,
      - T₉ identique ou différent de T₈, représente un atome d'hydrogène ou un radical (-CH₂-CH₂-O-)ₘ₉-H dans lequel m9 est un nombre entier supérieur ou égal à zéro et inférieur ou égal à dix, et
      - étant entendu que la somme m8 + m9 est supérieure à 0 et inférieure ou égale à dix ;
   **P) -** Optionnellement jusqu'à 40% molaire d'un composé de formule (V') 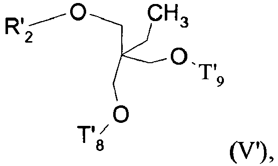 dans laquelle :
      - R'₂ représente un radical alkyle linéaire ou ramifié, comportant 14 atomes de carbone,
      - T'₈, représente un atome d'hydrogène ou un radical (-CH₂-CH₂-O-)ₘ₈-H dans lequel m8 est un nombre entier supérieur ou égal à zéro et inférieur ou égal à dix,
      - T'₉ identique ou différent de T'₈, représente un atome d'hydrogène ou un radical (-CH₂-CH₂-O-)ₘ₉-H dans lequel m9 est un nombre entier supérieur ou égal à zéro et inférieur ou égal à dix, et
      - étant entendu que la somme m8 + m9 est supérieure à 0 et inférieure ou égale à dix ; et
   γ) - Optionnellement jusqu'à 10% molaire d'un composé de formule 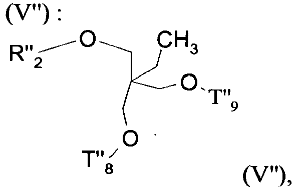 dans laquelle :
      - R"₂ représente un radical alkyle linéaire ou ramifié, comportant 16 atomes de carbone,
      - T"₈, représente un atome d'hydrogène ou un radical (-CH₂-CH₂-O-)ₘ₈-H dans lequel m8 est un nombre entier supérieur ou égal à zéro et inférieur ou égal à dix,
      - T"₉ identique ou différent de T"₈, représente un atome d'hydrogène ou un radical (-CH₂-CH₂-O-)ₘ₉-H dans lequel m9 est un nombre entier supérieur ou égal à zéro et inférieur ou égal à dix, et
      - étant entendu que la somme m8 + m9 est supérieure à 0 et inférieure ou égale à dix.

Selon un autre aspect particulier de la présente invention, ladite composition tensioactive (C) telle que définie précédemment, comprend en outre :
**4) -** Jusqu'à 5% molaire d'une composition (C_{VI}) comprenant pour 100% molaire :
   **α) -** De 60% molaire à 100% molaire d'un composé de formule (VI) :

      R2-(-CH₂-CH₂-O-)ₘ₁₀-H (VI),

      dans laquelle R2 représente un radical alkyle linéaire ou ramifié, comportant 12 atomes de carbone et m10 est un nombre entier supérieur ou égal à zéro et inférieur ou égal à dix ;
   **P) -** Optionnellement jusqu'à 40% molaire d'un composé de formule (VI') :

      R'₂-(-CH₂-CH₂-O-)ₘ₁₀-H (VI'),

      dans laquelle R'₂ représente un radical alkyle linéaire ou ramifié, comportant 14 atomes de carbone et m10 est un nombre entier supérieur ou égal à zéro et inférieur ou égal à dix ; et
   **γ) -** Optionnellement jusqu'à 10% molaire d'un composé de formule (VI") :

      R"₂-(-CH₂-CH₂-O-)ₘ₁₀-H (VI"),

      dans laquelle R"₂ représente un radical alkyle linéaire ou ramifié, comportant 16 atomes de carbone et m10 est un nombre entier supérieur ou égal à zéro et inférieur ou égal à dix.

Selon un aspect particulier de la présente invention, la dite composition tensioactive (C) telle que définie précédemment, comprend :
**1) -** Une proportion supérieure ou égale à 20% molaire et inférieure ou égale à 50% molaire d'une composition (C_{II}) telle que définie précédemment ;
**2) -** Une proportion supérieure ou égale à 50% molaire et inférieure ou égale à 80% molaire d'une composition (C_{II}) telle que définie précédemment.

Selon un autre aspect particulier de la présente invention :
**1)** - ladite composition (C_{II}) comprend pour 100% molaire :
   **α)** - De 60% à 80% molaire du composé de formule (II),
   **P) -** De 15% à 30% molaire du composé de formule (II'), et
   **γ**) - Jusqu'à 10% molaire du composé de formule (II"), et
**2) -** ladite composition (C_{III}) comprend pour 100% molaire :
   **α)** - De 60% à 80% molaire du composé de formule (III), de son isomère de formule (IV) ou du mélange de ces isomères,
   **P) -** de 15% à 30% molaire du composé de formule (III'), de son isomère de formule (IV') ou du mélange de ces isomères, et
   **γ)** - Jusqu'à 10% molaire du composé de formule (III"), de son isomère de formule (IV") ou du mélange de ces isomères.

Dans la composition telle que définie ci-dessus, le système émulsionnant (S₁) de type eau dans huile (E/H) est constitué soit d'un seul tensioactif soit d'un mélange de tensioactifs à condition que ledit tensioactif ou ledit mélange ait une valeur de HLB suffisamment faible pour induire des émulsions eau dans huile. Comme agent émulsionnant de type eau - dans huile, il y a par exemple les esters de sorbitan, comme l'oléate de sorbitan, comme celui commercialisé par la société SEPPIC sous le nom MONTANE™ 80, l'isostéarate de sorbitan, comme celui commercialisé par la société SEPPIC sous le nom MONTANE™ 70 ou le sesquioléate de sorbitan comme celui commercialisé par la société SEPPIC sous le nom MONTANE™ 83. Il y aussi certains esters de sorbitan polyéthoxylés, par exemple le mono-oléate de sorbitan pentaéthoxylé comme celui commercialisé par la société SEPPIC sous le nom MONTANOX™ 81 ou l'isostéarate de sorbitan pentaéthoxylé comme celui commercialisé sous le nom MONTANOX™ 71 par la société SEPPIC. Il y a encore l'alcool oléocétylique diéthoxylé, comme celui commercialisé sous le nom SIMULSOL™ OC 72 par la société SEPPIC, les polyesters de poids moléculaire compris entre 1000 et 3000, produits de la condensation entre un acide poly(isobutènyl) succinique ou son anhydride et tels que l'HYPERMER™ 2296 commercialisé par la société UNIQEMA ou enfin les copolymères blocks de poids moléculaire compris entre 2500 et 3500, comme l'HYPERMER™ B246 commercialisé par la société UNIQEMA ou le SIMALINE™ IE 200 commercialisé par la société SEPPIC.

Dans la composition objet de la présente invention, le système émulsionnant (S₂) de type huile dans eau (H/E) comprend au moins une quantité non nulle de la composition tensioactive (C) telle que définie ci-dessus. Par quantité non nulle, on désigne plus particulièrement une proportion supérieure ou égale à 10 % massique et, tout particulièrement supérieure ou égale à 50% massique.

Selon un mode particulier de la présente invention, ledit système émulsionnant (S₂) de type huile dans eau (H/E) consiste en 100% massique de la composition tensioactive (C) telle que définie précédemment.

Selon un autre mode particulier de la présente invention le système émulsionnant (S₂) de type huile dans eau (H/E) comprend en outre au moins un tensioactif émulsionnant du type (H/E) différent de l'un ou de l'autre des composés constituant ladite composition tensioactive (C) telle que définies précédemment.

Par "agent émulsionnant du type huile dans eau", on désigne des agents émulsionnants possédant une valeur de HLB suffisamment élevée pour fournir des émulsions huile dans eau, tels que :
- Les esters de sorbitan éthoxylés comme l'oléate de sorbitan polyéthoxylé avec 20 moles d'oxyde d'éthylène commercialisé par la société SEPPIC sous le nom de MONTANOX™ 80 ou le laurate de sorbitan polyéthoxylé avec 20 moles d'oxyde d'éthylène commercialisé par la société SEPPIC sous le nom de MONTANOX™ 20 ;
- L'huile de ricin polyéthoxylée avec 40 moles d'oxyde d'éthylène commercialisé sous le nom SIMULSOL™ OL50 ;
- L'alcool oléodécylique décaéthoxylé, commercialisé par la société SEPPIC sous le nom SIMULSOL™ OC 710 ;
- L'alcool laurique heptaéthoxylé commercialisé sous le nom SIMULSOL™ P7 ;
- Ou les hexaoléates de sorbitan polyéthoxylés commercialisés par la société SEPPIC sous le nom SIMALINE™ IE 400.

Selon un mode particulier de la composition telle que définie précédemment, le système émulsionnant (S₂) de type huile dans eau (H/E) comprend en outre une proportion massique non nulle d'au moins un agent émulsionnant du type huile dans eau choisi parmi l'oléate de sorbitan polyéthoxylé avec 20 moles d'oxyde d'éthylène ; le laurate de sorbitan polyéthoxylé avec 20 moles d'oxyde d'éthylène ; l'huile de ricin polyéthoxylée avec 40 moles d'oxyde d'éthylène ; l'alcool oléodécylique décaéthoxylé, l'alcool laurique heptaéthoxylé ; ou les hexaoléates de sorbitan polyéthoxylés.

Selon un mode tout particulier de la présente invention, ledit système émulsionnant (S₂) de type huile dans eau (H/E) comprend pour 100% de sa masse :
- de 10% massique à 40% massique d'alcool laurique heptaéthoxylé et
- de 60% massique à 90% massique de ladite composition tensioactive (C).

Par polyélectrolyte branché, on désigne pour (P), un polyélectrolyte non linéaire qui possède des chaînes pendantes de manière à obtenir, lorsque ce polyélectrolyte est mis en solution dans l'eau, un fort état d'enchevêtrement conduisant à des viscosités à bas gradient très importantes.

Par polyélectrolyte réticulé on désigne pour (P), un polyélectrolyte non linéaire se présentant à l'état de réseau tridimensionnel insoluble dans l'eau, mais gonflable à l'eau et conduisant donc à l'obtention d'un gel chimique.

La composition selon l'invention peut comporter des polyélectrolytes réticulés et/ou des polyélectrolytes branchés.

Lorsque le polyélectrolyte (P) est réticulé, il l'est plus particulièrement avec un composé diéthylénique ou polyéthylènique dans une proportion molaire exprimée par rapport aux monomères mis en oeuvre dépendant la nature chimique du réticulant et généralement inférieure ou égale à 0,40% molaire, principalement inférieure à 0,25% molaire, plus particulièrement inférieure ou égale à 0,05% molaire et tout particulièrement comprise entre 0,005 % et 0,01 % molaire. De préférence, l'agent de réticulation et/ou l'agent de ramification est choisi parmi le diméthacrylate d'éthylèneglycol, le diacrylate de diéthylèneglycol, le diallyloxyacétate de sodium. le diacrylate d'éthylèneglycol, le diallyl urée, le triallylamine, le triméthylol propanetriacrylate, le méthylène-bis(acrylamide) ou un mélange de ces composés.

L'invention a plus particulièrement pour objet, une composition telle que définie précédemment, pour laquelle dans le polyélectrolyte (P), l'unité monomérique issue du monomère de formule (I) est une unité monomérique issus de l'acrylate de lauryle tétraéthoxylé.

Le polyélectrolyte (P) est alors de préférence choisi parmi :
- Les copolymères réticulés de l'acide acrylique partiellement salifié sous forme de sel de sodium ou de sel d'ammonium, de l'acrylamide et de l'acrylate de lauryle tétraéthoxylé ;
- Les copolymères réticulés de l'acide 2-méthyl 2-[(1-oxo 2-propènyl) amino] 1-propanesulfonique partiellement salifié sous forme de sel de sodium ou de sel d'ammonium, de l'acrylamide et de l'acrylate de lauryle tétraéthoxylé ;
- Les copolymères réticulés de l'acide 2-méthyl 2-[(1-oxo 2-propènyl) amino] 1-propanesulfonique partiellement salifié sous forme de sel de sodium ou de sel d'ammonium, de l'acrylate de 2-hydroxy éthyle et de l'acrylate de lauryle tétraéthoxylé ;
- Les copolymères réticulés de l'acrylamide, de l'acide 2-méthyl 2-[(1-oxo 2-propènyl) amino] 1-propanesulfonique, de l'acide acrylique partiellement salifiés sous forme de sel sodium ou de sel d'ammonium et de l'acrylate de lauryle tétraéthoxylé ;
- Les copolymères de l'acide 2-méthyl 2-[(1-oxo 2-propènyl) amino] 1-propanesulfonique partiellement salifié sous forme de sel de sodium ou de sel d'ammonium, de l'acrylamide, du vinyl pyrrolidone et de l'acrylate de lauryle tétraéthoxylé ; et
- Les copolymères réticulés de l'acide 2-méthyl 2-[(1-oxo 2-propènyl) amino] 1-propanesulfonique partiellement ou totalement salifié sous forme de sel de sodium, de l'acide acrylique partiellement salifiés sous forme de sel sodium ou de sel d'ammonium, de l'acrylate de 2-hydroxy éthyle, de tris(hydroxyméthyl)aminométhyl acrylamide et de l'acrylate de lauryle tétraéthoxylé.

Selon un autre aspect particulier de la présente invention, le polyélectrolyte anionique (P) réticulé, comporte pour 100% de monomères mis en oeuvre :
- De 20% molaire à 80% molaire d'unités monomériques issues d'un monomère comportant soit une fonction acide fort, soit une fonction acide faible ;
- De 15% molaire à 75% molaire d'unités monomériques issues d'un monomère neutre différent du composé de formule (I) telle que définie précédemment ;
- De 0,5% à 5% molaire d'unités monomériques issues d'un monomère de formule (I) telle que définie précédemment.

Selon un autre aspect particulier de la présente invention, le polyélectrolyte anionique (P) réticulé comporte pour 100% de monomères mis en oeuvre :
- De 40% molaire à 80% molaire d'unités monomériques issues d'un monomère comportant une fonction acide fort ;
- De 15% molaire à 55% molaire d'unités monomériques issues d'un monomère neutre différent du composé de formule (I) telle que définie précédemment ;
- De 1% à 5% molaire d'unités monomériques issues d'un monomère de formule (I) telle que définie précédemment.

Dans la composition objet de la présente invention, la phase huile est constituée soit par une huile minérale commerciale contenant des hydrocarbures saturés comme les paraffines, les isoparaffines ou les cycloparaffines présentant à température ambiante, une densité entre 0,7 et 0,9 et un point d'ébullition supérieur à environ 250°C, telle que par exemple le MARCOL™ 52 ou le MARCOL™ 82, commercialisés par EXXON CHEMICAL, soit par une huile végétale comme le squalane d'origine végétale, soit par une huile de synthèse tel que le polyisobutène hydrogéné ou le polydécène hydrogéné, soit par un mélange de plusieurs de ces huiles. Le MARCOL™ 52 est une huile commerciale répondant à la définition des huiles de vaseline du Codex français. C'est une huile blanche minérale conforme aux réglementations FDA 21 CFR 172.878 et CFR 178.3620 (a) et elle est inscrite à la Pharmacopée des USA, US XXIII (1995) et à la Pharmacopée européenne (1993). La composition selon l'invention peut également contenir divers additifs tels que des agents complexants, des agents de transfert ou des agents limiteurs de chaîne.

Selon un autre aspect de la présente invention, celle-ci a pour objet un procédé de préparation de la composition telle que définie précédemment, caractérisé en ce que :
a) l'on émulsionne une phase aqueuse (A) contenant les monomères et les éventuels additifs hydrophiles, dans une phase organique (O) contenant, le système tensioactif (S₁), un mélange constitué de l'huile destinée à être présente dans la composition finale et d'une huile volatile et les éventuels additifs hydrophobes,
b) l'on amorce la réaction de polymérisation par introduction dans l'émulsion formée en a), d'un initiateur de radicaux libres puis on la laisse se dérouler, et
c) l'on concentre par distillation le milieu réactionnel issu de l'étape b), jusqu'à élimination complète de ladite huile volatile ;
d) l'on introduit dans le milieu concentré issu de l'étape c), ledit système émulsionnant (S₂) de type huile dans eau (H/E) à une température inférieure ou égale à 70°C.

Les huiles volatiles appropriées à la mise en oeuvre du procédé tel que défini ci- dessus, sont par exemple des isoparaffines légères comportant de 8 à 13 atomes de carbone comme par exemple celles vendues sous les noms ISOPAR™ G, ISOPAR™ L ou ISOPAR™ H ou ISOPAR™ J.

Selon une mise en oeuvre préférée du procédé tel que défini précédemment, la réaction de polymérisation est amorcée par un couple oxydoréducteur, tel que le couple hydroperoxyde de cumène - métabisulfite de sodium, à une température inférieure ou égale à 10°C, puis conduite soit de manière quasi-adiabatique jusqu'à une température supérieure ou égale à 40°C, plus particulièrement supérieure ou égale à 50°C, soit en contrôlant l'évolution de la température.

Le procédé de préparation de ladite composition tensioactive (C) telle que définie précédemment comprend les étapes successive suivantes :
**Une étape a)** de réaction d'un mélange d'alcools comprenant pour 100% molaire :
   - De 60% molaire à 100% molaire d'un composé de formule (VII),

      R2-O-H (VII),

      dans laquelle R2 représente un radical alkyle linéaire ou ramifié, comportant 12 atomes de carbone ;
   - Optionnellement jusqu'à 40% molaire d'un composé de formule (VII') :

      R'₂-O-H (VII'),

      dans laquelle R'₂ représente un radical alkyle linéaire ou ramifié, comportant 14 atomes de carbone, et
   - Optionnellement jusqu'à 10% molaire d'un composé de formule (VII") :

      R"₂-O-H (VII"),

      dans laquelle R"₂ représente un radical alkyle linéaire ou ramifié, comportant 16 atomes de carbone;
avec un excès de 3-(hydroxyméthyl) 3-éthyl oxétane de formule (VIII) : 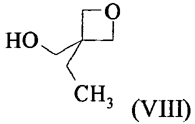 pour former une composition (C') comprenant :
**α)** - Le composé de formule (IX) : 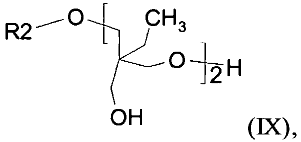 dans laquelle R2 est tel que défini ci-dessus ;
**β)** - Optionnellement le composé de formule (IX)' : 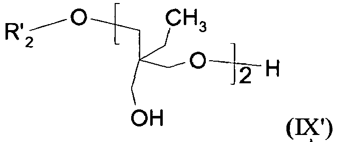 dans laquelle R'₂ est tel que défini ci-dessus ;
**γ) -** Optionnellement le composé de formule (IX") : 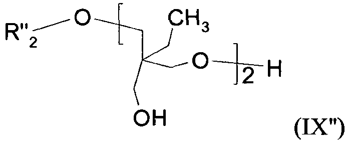 dans laquelle R"₂ est tel que défini ci-dessus ;
**δ)** - Le composé de formule (X) : 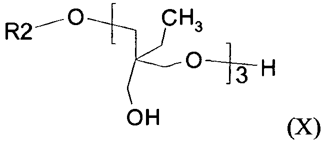 ou son isomère de formule (XI) : 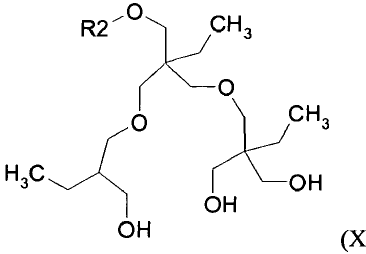 ou un mélange de ces deux isomères ; composés de formules (X) et (XI) dans lesquelles R2 est tel que défini ci-dessus ;
**ε)** - Optionnellement le composé de formule (X') : 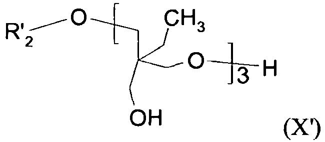 ou son isomère de formule (XI') : 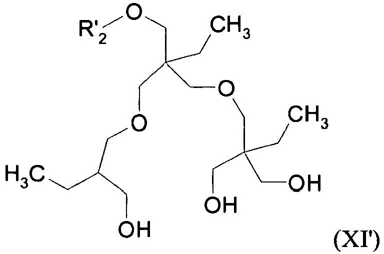 ou un mélange de ces deux isomères ; composés de formules (X') et (XI') dans lesquelles R'₂ est tel que défini ci-dessus ;
**ζ)** - Optionnellement le composé de formule (X") : 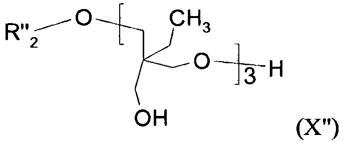 ou son isomère de formule (XI") : 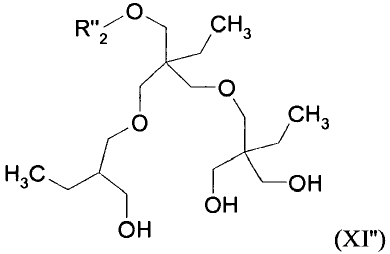 ou un mélange de ces deux isomères ; composés de formules (X") et (XI") dans lesquelles R"₂ est tel que défini ci-dessus ;

**Une étape b)** de réaction de ladite composition (C') avec l'oxyde d'éthylène de formule (XII) : 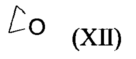 pour former ladite composition tensioactive (C) telle que définie précédemment.

L'invention a aussi pour objet l'utilisation de la composition telle que définie précédemment, comme agent épaississant et/ou émulsionnant, pour préparer une composition topique cosmétique, dermopharmaceutique ou pharmaceutique.

L'invention a aussi pour objet une composition topique cosmétique, dermopharmaceutique ou pharmaceutique, caractérisée en ce qu'elle comprend, comme agent épaississant et/ou émulsionnant, une quantité efficace de la composition telle que définie précédemment.

Une composition topique selon l'invention, destinée à être appliquée sur la peau, sur le cuir chevelu ou les muqueuses de l'homme ou de l'animal, peut consister en une émulsion topique comprenant au moins une phase aqueuse et au moins une phase huile, se présentant sous la forme d'une émulsion eau dans huile, ou huile dans eau, ou eau dans huile dans eau, ou huile dans eau dans huile. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un myorelaxant, un antifongique ou un antibactérien.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un antirides, un agent à visée amincissante, un agent antiradicalaire, un agent antipelliculaire, un agent anti-acnéique ou un antifongique.

Par quantité efficace, on signifie que la composition topique selon l'invention comporte une quantité suffisante de latex inverse selon l'invention pour provoquer une modification de sa rhéologie. La composition topique selon l'invention comporte habituellement entre 0,1% et 10% en poids dudit latex inverse auto-inversible défini ci-dessus. Le pH de la composition topique est généralement compris entre 3 et 9.

La composition topique peut en outre comporter des composés classiquement compris dans ce type de compositions, par exemple des parfums, des conservateurs, des colorants, des émollients ou des tensioactifs.

Selon encore un autre aspect, l'invention concerne l'utilisation du nouvel agent épaississant et/ou émulsionnant conforme à l'invention, mentionné ci-dessus, pour épaissir et émulsionner une composition topique comprenant au moins une phase aqueuse.

La composition selon l'invention est un substitut intéressant à celles vendues sous les noms SEPIGEL™ 305, SEPIGEL™ 501, SIMULGEL™ EG, SIMULGEL™ NS ou SIMUL-GEL™ 600 par la demanderesse, car elle présente aussi une bonne compatibilité avec les autres excipients utilisés pour la préparation de formulations telles que les laits, les lotions, les crèmes, les savons, les bains, les baumes, les shampooings ou les après- shampooings. Elle peut aussi être mise en oeuvre avec lesdits SEPIGEL ou SIMULGEL. Elle est notamment compatible avec les concentrés décrits et revendiqués dans les publications internationales WO 92/06778, WO 95/04592, WO 95/13863, WO 96/37285, WO 98/22207, WO 98/47610 ou dans FR 2734 496, avec les agents tensioactifs décrits dans WO 93/08204. Elle est particulièrement compatible avec le MONTANOV™ 68, le MONTANOV™ 82, le MONTANOV™ 202, le MONTANOV™ L, le MONTANOV™ 14 ou le MONTANOV™S. Elle peut également être utilisée dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316. Elle peut également être utilisée pour former des gels aqueux à pH acide cosmétiquement ou physiologiquement acceptable, tels que ceux décrit dans WO 93/07856 ; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019 ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 195 23596 ou en association avec d'autres polymères épaississants.

La composition selon l'invention est également compatible avec les principes actifs tels que par exemple, les agents auto-bronzants comme le dihydroxyacétone (DHA) ou les agents anti-acné ; elle peut donc être introduite dans des compositions auto-bronzantes comme celles revendiquées dans EP0 715 845, EP 0 604 249, EP 0 576 188 ou dans 93/07902.

Elle est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou dans WO 98/09611, et ce qui permet également son utilisation dans des compositions éclaircissantes de la peau humaine telles que celles décrites ou revendiquée dans WO2003/061768.

Lorsque la composition telle que définie précédemment est destinée au traitement de la peau et/ou du cuir chevelu et/ou des muqueuses, elle comprend plus particulièrement un latex inverse de polyélectrolyte anionique objet de la présente invention. Les latex inverse objet de la présente invention peuvent être utilisés comme épaississant de pâtes d'impression textile.

Les exemples qui suivent ont pour but d'illustrer la présente invention sans toutefois la limiter.

### Exemple A) : Préparation d'une composition tensioactive (C) mise en oeuvre dans la composition objet de la présente invention

### Stade A1) : Préparation de la composition intermédiaire (C')

21.100 g d'un mélange d'alcools gras comprenant de 65% à 75% massique d'alcanol comportant 12 atomes de carbone, de 21% à 28% massique d'alcanol comportant 14 atomes de carbone et de 4% à 8% massique d'alcanol comportant 16 atomes de carbone, préalablement chauffés, sont introduits dans un réacteur, maintenus sous agitation et séchés, On ajoute ensuite 326 grammes de trifluorure de bore à 50% dans le diéthyl-éther puis progressivement sous agitation pendant 4 heures, 32.600 g de 3-(hydroxyméthyl) 3-éthyl oxétane, en maintenant la température autour de 110°C. Le milieu réactionnel est alors laissé encore 11 heures à 115°C. On obtient alors la composition (C') attendue, caractérisée comme suit :
- Aspect à 25°C : Gel trouble
- Indice d'acide (en mg KOH/g ; NFT60-204) : 3,9
- Indice d'hydroxyle (en mg KOH/g) : 400,5
- Teneur massique en 3-(hydroxyméthyl) 3-éthyl oxétane libre (déterminée par chromatographie en phase gazeuse) : < 0.05%
- Teneur massique en alcanols libres (déterminée par chromatographie en phase gazeuse) : alcanol en C12 : 5,7% ; alcanol en C14 : 2,0% ; alcanol en C16 : 0,5%.

### Stade A2) : Préparation de la composition tensioactive (C)

50.000 g de la composition intermédiaire (C') obtenue au stade A1) précédent, sont introduits dans un autoclave d'une capacité de 0,1 m³, avec 75 g de potasse puis séchés à une température de 105°C. Une quantité de 35 000 g d'oxyde d'éthylène est ensuite progressivement introduite en régulant la température du mélange réactionnel à une valeur de 125°C. Une fois la quantité totale d'oxyde d'éthylène introduite, le mélange réactionnel est maintenu sous agitation à 125°C pendant une durée supplémentaire d'une heure. Le produit alors obtenu est ensuite refroidi à une température de 80°C et vidangé. On obtient alors la composition tensioactive (C) caractérisée comme suit :
Aspect à 30°C : liquide limpide
Couleur : 125 Alpha
Indice d'hydroxyle (en mg KOH/g) : 252,5
Indice d'acide (en mg KOH/g) (NFT60-204) : 0,08
Teneur résiduelle en eau : 0.05%
Point de trouble (NF EN 1890E) : 76°C
Teneur massique en alcanols libres (chromatographie en phase gazeuse) : Alcanol en C12 : 1,2% ; alcanol en C14 : 0,4% ; alcanol en C16 : 0,1% soit au total 1.7% d'alcanols résiduels
Viscosité à 25°C (Brookfield LVT Mobile 3 Vitesse 12) : 1.072 mPa.s

### EXEMPLE 1 (selon l'invention) : Latex inverse auto-inversible du copolymère ATBS(sel de Na) / HEA / (ALE-4 OE) [(ATBS/HEA/(ALE-4OE) 89,0/9,9/1,1 molaire] réticulé au MBA

### 1) Préparation

a) - Dans un premier bêcher, on introduit successivement sous agitation :
   - 672,5g d'une solution commerciale à 55% massique de sel de sodium de l'acide 2-acrylamido 2-méthyl propanesulfonique (ATBS Na),
   - 20,8g d'acrylate de 2-hydroxy éthyle (HEA) ;
   - 0,028g de méthylène bis(acrylamide) (MBA) ;et
   - 1,0 g d'une solution commerciale à 40% massique de diéthylènetriaminepenta acétate de sodium.
      Puis on y ajuste le pH à 4 en ajoutant si nécessaire la quantité requise d'acide 2-acrylamido 2-méthyl propane sulfonique et de l'eau permutée jusqu'à 700 g
**b) -** Dans un deuxième bêcher, on introduit successivement sous agitation :
   130 g de polyisobutène,
   30 g de MARCOL™ 52,
   90 g d'ISOPAR™ H,
   17 g de MONTANE™ 70,
   3 g d'HYPERMER™ 6212,
   5 g de SIMALINE™IE 200,
   7,2 g d'acrylate de lauryle tétraéthoxylé (commercial) (ALE-4OE),
   0,36g de peroxyde de dilauroyle.
**c) -** La phase aqueuse est alors incorporée dans la phase organique sous agitation et puis la pré-émulsion ainsi obtenue est soumise à une agitation mécanique cisaillante à l'aide d'une turbine de type Silverson de manière à créer une émulsion fine sous barbotage d'azote.
**d) -** Après refroidissement à environ 8 °C, la réaction de polymérisation est initiée à l'aide du couple oxydoréducteur : hydroperoxyde de cumène / métabisulfite de sodium.
**e) -** Une fois la réaction de polymérisation terminée, on retire par distillation sous vide l'ISOPAR™ H et la quasi-totalité de l'eau.
**f) -** On obtient après introduction de 2% massique de Laureth-7 et de 4% massique de la composition tensioactive (C) obtenue à l'exemple A, le **latex inverse auto-inversible (1)** contenant environ 63% de polymère, qui est peu visqueux, qui s'inverse très rapidement dans l'eau et dont le pouvoir épaississant est important. De plus ce latex inverse est très stable car on n'observe aucun phénomène de synérèse, en ce qu'il ne relargue que de très peu d'huile et que polymère ne sédimente pas. Sa teneur en eau mesurée par une titrimétrie Karl-Fisher est de 1,8% en poids.

### 2) Mesures de viscosité

**a)** - On mesure la viscosité du latex inverse auto-inversible (1) obtenu comme indiqué au paragraphe 1, celle d'une solution aqueuse exempte de chlorure de sodium (Sol.1) et celles de solutions aqueuses contenant respectivement 0,1 % massique (Sol.2) et 1% massique (Sol.3) de chlorure de sodium, lesdites solutions aqueuses comprenant chacune 2% massique dudit latex inverse auto-inversible (1). Les résultats mesurés à l'aide d'un viscosimètre Brookfield RVT sont consignés dans le tableau suivant :

| | Mobile (M) ; Vitesse de rotation du mobile (V) (en tours par minute) | Viscosité (en mPa.s) |
|---|---|---|
| Latex inverse (1) | M3, V20 | 2 700 |
| Sol.1 | M6, V5 | 54 000 |
| Sol. 2 | M6, V5 | 27 000 |
| Sol.3 | M3, V5 | 1 600 |

### 3) Mesure du temps d'inversion et évaluation de la stabilité du latex inverse.

**a) -** Le temps d'inversion est évalué en mesurant le temps nécessaire pour obtenir un gel lisse et homogène pour une solution aqueuse à 2% massiques de latex inverse auto-inversible (1) dans les conditions standard de mesure de cette viscosité c'est-à-dire en incorporant 16 g du latex inverse (1) dans 784 g d'eau, le tout étant placé dans un bécher de 1 litre de forme basse, puis en agitant le tout à l'aide d'une hélice type papillon tournant à 150 tours par minute. Le temps d'inversion est donc la durée évaluée entre la mise en route de l'agitateur et l'apparition d'un milieu lisse et homogène dans le bêcher. Dans le présent exemple, le temps d'inversion est de 50 secondes.
**b) -** La stabilité du latex inverse est évaluée en observant le temps d'apparition d'une couche huile en surface. Dans le présent exemple, le temps d'apparition de la couche d'huile en surface du latex inverse (1) est de deux semaines.

### EXEMPLE T1 (selon l'état de la technique) : Latex inverse auto-inversible du copolymère ATBS(sel de Na) / HEA / (ALE-4OE) [(ATBS/HEA/(ALE-4OE) 89,0/9,9/1,1 molaire] réticulé au MBA

### 1) Préparation

On reproduit les étapes a) à d) de l'exemple 1. A l'étape f), on ajoute 4% massique de MONTANOX™ 20 à la place de 4% massique de la composition tensioactive (C) et l'on obtient le latex inverse auto-inversible (T1).

### 2) Mesures de viscosité

**a) -** On mesure la viscosité du latex inverse auto-inversible (T1) obtenu comme indiqué au paragraphe 1, celle d'une solution aqueuse exempte de chlorure de sodium (Sol.4) et celles de solutions aqueuses contenant respectivement 0,1% massique (Sol.5) et 1% massique (Sol.6) de chlorure de sodium, lesdites solutions aqueuses comprenant chacune 2% massique dudit latex inverse auto-inversible (T1). Les résultats mesurés à l'aide d'un viscosimètre Brookfield RVT sont consignés dans le tableau suivant :

| | Mobile (M) ; Vitesse de rotation du mobile (V) (en tours par minute) | Viscosité (en mPa.s) |
|---|---|---|
| Latex inverse (T1) | M3, V20 | 2.900 |
| Sol.4 | M6, V5 | 51.200 |
| Sol. 5 | M6, V5 | 25.200 |
| Sol.6 | M3, V5 | 1.300 |

### 3) Mesure du temps d'inversion et évaluation de la stabilité du latex inverse.

**a)** - Le temps d'inversion évalué de la même manière qu'à l'exemple précédent est de 2 minutes 20 secondes.
**b) -** La stabilité du latex inverse (T1) est évaluée de la même manière qu'à l'exemple précédent. Un relargage important d'huile est observé au bout d'une semaine.

### EXEMPLE 2 (selon l'invention) : Latex inverse auto-inversible du copolymère ATBS(sel de Na) / HEA / (ALE-4OE) [(ATBS/HEA/(ALE-4OE) 89,0/9,9/1,1 molaire] réticulé au MBA

### 1) Préparation

On reproduit les étapes a) à d) de l'exemple 1. A l'étape f), on n'ajoute que 4% massique de la composition tensioactive (C) et l'on obtient le latex inverse auto-inversible (2).

### 2) Viscosimétrie, mesure du temps d'inversion et évaluation de la stabilité du latex inverse (2)

**a)** - Les performances viscosimétriques du latex inverse (2) sont similaires à celles rapportées pour le latex inverse de l'exemple 1.
**b) -** Le temps d'inversion du latex inverse (2), évalué de la même manière qu'à l'exemple 1, est d'environ 40 secondes.
**c) -** La stabilité du latex inverse (2) est évaluée de la même manière qu'à l'exemple 1. Le temps d'apparition de la couche d'huile est de quelques jours.

### EXEMPLE T2 (selon l'état de la technique) : Latex inverse auto-inversible du copolymère ATBS(sel de Na) / HEA / (ALE-4OE) [(ATBS/HEA/(ALE4OE) 89,0/9,9/1,1 molaire] réticulé au MBA

### 1) Préparation

On reproduit les étapes a) à d) de l'exemple 1. A l'étape f), on ajoute 4% massique d'une composition (C"'), qui comprend pour 100% molaire :
i) - Une proportion supérieure ou égale à 10% molaire et inférieure ou égale à 50% molaire d'un composé de formule (II"') correspondant à la formule (II) dans laquelle R2 représente un radical alkyle linéaire ou ramifié, comportant 10 atomes de carbone, et dans la quelle la somme m1 + m2 + m3 est égale à 5;
ii) - Une proportion supérieure ou égale à 50% molaire et inférieure ou égale à 90% molaire d'un composé de formule (III"') ou de son isomère de formule (IV"') ou du mélange de ces deux isomères, formules (III"') et (IV"') correspondant respectivement aux formules (III) et (IV) dans lesquelles R2 représente un radical alkyle linéaire ou ramifié, comportant 10 atomes de carbone, et dans la quelle la somme m4 + m5 + m6 + m7 est égale à 5; et l'on obtient le latex inverse auto-inversible (T2).

### 2) Viscosimétrie, mesure du temps d'inversion et évaluation de la stabilité du latex inverse (T2)

**a) -** Les performances viscosimétriques du latex inverse (T2) sont similaires à celles rapportées pour le latex inverse de l'exemple 1.
b) - Le temps d'inversion du latex inverse (T2), évalué de la même manière qu'à l'exemple 1, est d'environ 50 secondes.
c) - La stabilité du latex inverse (T2) est évaluée de la même manière qu'à l'exemple 1. Le temps d'apparition de la couche d'huile est de quelques heures.

### EXEMPLE T3 (selon l'état de la technique) : Latex inverse auto-inversible du copolymère AM / AA / (ALE-4OE) [(AM/AA/(ALE-4OE) 24,7/74,1/1,2 molaire] réticulé au MBA

### 1) Préparation

**a)** - Dans un premier bêcher, on introduit successivement sous agitation :
   - 106,5 g d'une solution commerciale d'acrylamide (AM) à 50% (massique)
   - 162,0 g d'acide acrylique glacial (AA),
   - 98,1 g d'une solution d'ammoniaque à 29,3% en poids,
   - 0,047 g de méthylène bis(acrylamide) (MBA).
   - 0,45 g d'une solution commerciale à 40% du diéthylènetriaminepentaacétate de sodium,
   - de l'eau permutée jusqu'à 680 g.
**b) -** Dans un deuxième bêcher, on introduit successivement sous agitation :
   - 121 g de polyisobutène,
   - 28 g de MARCOL™ 52,
   - 99 g d'ISOPAR™ H,
   - 17 g de MONTANE™ 70,
   - 3 g d'HYPERMER™ 2296,
   - 5 g de SIMALINE™IE 200,
   - 1,2 g d'acrylate de lauryle tétraéthoxylé (commercial) (ALE-4OE),
   - 0,1 g d'AIBN.
**c) -** La phase aqueuse est alors incorporée dans la phase organique sous agitation et puis la pré-émulsion ainsi obtenue est soumise à une agitation mécanique cisaillante à l'aide d'une turbine de type Silverson de manière à créer une émulsion fine sous barbotage d'azote.
**d) -** Après refroidissement à environ 8 °C, la réaction de polymérisation est initiée à l'aide du couple oxydoréducteur : hydroperoxyde de cumène / métabisulfite de sodium.
**e)** - Une fois la réaction de polymérisation terminée, on retire par distillation sous vide l'ISOPAR™ H et la quasi-totalité de l'eau.
**f) -** On obtient après introduction de 4% de MONTANOX™ 20 et de 2% de Laureth 7, le latex inverse auto-inversible (T3), contenant environ 63% de polymère, qui est peu visqueux, qui s'inverse très rapidement dans l'eau et dont le pouvoir épaississant est important. Sa teneur en eau mesurée par une titrimétrie Karl-Fisher est de 1,8% en poids.

### 2) Mesures de viscosité

**a) -** On mesure la viscosité du latex inverse auto-inversible (T3) obtenu comme indiqué au paragraphe 1, celle d'une solution aqueuse exempte de chlorure de sodium (Sol.7) et celles de solutions aqueuses contenant respectivement 0,1 % massique (Sol.8) et 1% massique (Sol.9) de chlorure de sodium, lesdites solutions aqueuses comprenant chacune 2% massique dudit latex inverse auto-inversible (T3). Les résultats mesurés à l'aide d'un viscosimètre Brookfield RVT sont consignés dans le tableau suivant :

| | Mobile (M) ; Vitesse de rotation du mobile (V) (en tours par minute) | Viscosité (en mPa.s) |
|---|---|---|
| Latex inverse (T3) | | nd |
| Sol.7 | M6, V5 | 79 400 |
| Sol. 8 | M6, V5 | 45 200 |
| Sol.9 | M3, V5 | 3 300 |

| | | |
|---|---|---|
| nd : non déterminée | | |

### 3) Mesure du temps d'inversion et évaluation de la stabilité du latex inverse (T3).

**b) -** Le temps d'inversion du latex inverse (T3), évalué de la même manière qu'à l'exemple 1, est d'environ 2 minutes.
**c) -** La stabilité du latex inverse du latex inverse (T3) est évaluée de la même manière qu'à l'exemple 1. Le temps d'apparition de la couche d'huile est de deux semaines.

### EXEMPLE 3 (selon l'invention) : Latex inverse auto-inversible du copolymère AM / AA / (ALE-4OE) [(AM/AA/(ALE-4OE) 24,7/74,1/1,2 molaire] réticulé au MBA

### 1) Préparation

On reproduit les étapes a) à d) de l'exemple T3. A l'étape f), on ajoute 2% massique de Laureth 7 et 4% massique de la composition tensioactive (C) au lieu des de 4% de MONTA-NOX™ 20 et 2% de Laureth 7 dudit exemple T3 et l'on obtient le latex inverse auto-inversible (3).

### 2) Viscosimétrie, mesure du temps d'inversion et évaluation de la stabilité du latex inverse (3)

**a)** - Les performances viscosimétriques du latex inverse (3) sont similaires à celles rapportées pour le latex inverse de l'exemple T3.
**b) -** Le temps d'inversion du latex inverse (3), évalué de la même manière qu'à l'exemple 1, est d'environ 30 secondes.
**c) -** La stabilité du latex inverse (3) est évaluée de la même manière qu'à l'exemple 1. Le temps d'apparition des premières gouttes d'huile est de trois semaines.

### EXEMPLE 4 (selon l'invention) : Latex inverse auto-inversible du copolymère ATBS (sel de Na) / AA / HEA / THAM / (ALE-4OE) [ATBS/AA/HEA/THAM(ALE-4OE) 83,9/1.9/9,3/3,7/1,2 molaire] réticulé au MBA

### 1) Préparation

**a) -** Dans un premier bêcher, on introduit successivement sous agitation :
   - 672,5 g d'une solution commerciale à 55% (massique) du sel de sodium de l'acide 2 acrylamido 2 méthyl propanesulfonique (ATBSNa) ;
   - 20,8 g d'acrylate de 2-hydroxy éthyle ;
   - 12,6g de THAM;
   - 2,6 g d'acide acrylique (AA)
   - 0,041 g de méthylène bis(acrylamide) (MBA).
   - 0,45 g d'une solution commerciale à 40% du diéthylènetriaminepentacétate de sodium,

   Puis on y ajuste le pH à 4 en ajoutant si nécessaire la quantité requise d"acide 2 acrylamido 2 méthyl propane sulfonique et de l'eau permutée jusqu'à 700 g
**b) -** Dans un deuxième bêcher, on introduit successivement sous agitation :
   - 130 g de polyisobutène,
   - 30 g de MARCOL™ 52,
   - 90 g d'ISOPAR™ H,
   - 17 g de MONTANE™ 70,
   - 5 g d'HYPERMER™ 6212,
   - 3g de DEHYMULS PGPH (Polyhydroxy stéarate de polyglycérol),
   - 7,4 g d'acrylate de lauryle tétraéthoxylé (commercial) (ALE-4OE),
   - 0,14g de dilauroyle peroxyde
c) - La phase aqueuse est alors introduite dans la phase organique sous agitation et puis la pré-émulsion ainsi obtenue est soumise à une agitation mécanique cisaillante à l'aide d'une turbine de type Silverson de manière à créer une émulsion fine sous barbotage d'azote.
d) - Après refroidissement à environ 8 °C, la réaction de polymérisation est initiée à l'aide du couple oxydoréducteur : hydroperoxyde de cumène / métabisulfite de sodium.
e) - Une fois la réaction de polymérisation terminée, on retire par distillation sous vide l'ISOPAR™ H et la quasi-totalité de l'eau.
f) - On obtient après introduction de 2% massique de Laureth-7 et de 4% massique de la composition tensioactive (C) obtenue à l'exemple A, le latex inverse auto-inversible (4), contenant environ 63% de polymère, qui est peu visqueux, qui s'inverse très rapidement dans l'eau et dont le pouvoir épaississant est important. Sa teneur en eau mesurée par une titrimétrie Karl-Fisher est de 2,2% en poids.

### 2) Mesures de viscosité

**a) -** On mesure la viscosité du latex inverse auto-inversible (4) obtenu comme indiqué au paragraphe 1, celle d'une solution aqueuse exempte de chlorure de sodium (sol.10) et celle d'une solution aqueuse contenant 0,1% massique (Sol.11) de chlorure de sodium, lesdites solutions aqueuses comprenant chacune 2% massique dudit latex inverse auto-inversible (4). Les résultats mesurés à l'aide d'une viscosimètre Brookfield RVT sont consignés dans le tableau suivant :

| | Mobile (M) ; Vitesse de rotation du mobile (V) (en tours par minute) | Viscosité (en mPas) |
|---|---|---|
| Latex inverse (4) | M3, V20 | 1.100 |
| Sol.10 | M6, V5 | 66.200 |
| Sol.11 | M6, V5 | 16.500 |

| | | |
|---|---|---|
| nd : non déterminée | | |

### 3) Mesure du temps d'inversion et évaluation de la stabilité du latex inverse.

**b) -** Le temps d'inversion du latex inverse (4), évalué de la même manière qu'à l'exemple 1, est d'environ 30 secondes.
**c) -** La stabilité du latex inverse (4) est évaluée de la même manière qu'à l'exemple 1. Le temps d'apparition des premières gouttes d'huile est de trois semaines.

### Exemples de formulations cosmétiques (proportions exprimées en pourcentages massiques)

### Exemple 5 Crème de soin

| | |
|---|---|
| Cyclométhicone : | 10% |
| Latex inverse auto-inversible (1) : | 0,8% |
| MONTANOV™ 68 : | 4,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | qsp 100% |

### Exemple 6 : Crème de soin

| | |
|---|---|
| Cyclométhicone : | 10% |
| Latex inverse auto-inversible (3) : | 0,8% |
| MONTANOV™ 68: | 4,5% |
| Perfluoropolyméthylisopropyléther : | 0,5% |
| Conservateur : | 0,65% |
| Lysine : | 0,025% |
| EDTA (sel disodique) : PEMULEN™ TR : | 0,05% |
| Glycérine : | 3% |
| Eau: | qsq 100% |

### Exemple 7 : Baume après-rasage

**FORMULE**

| | | |
|---|---|---|
| A | Latex inverse auto-inversible (2) : | 1,5% |
| | Eau : | qsq. 100% |
| B | MICROPEARL™ M100 : | 5,0% |
| | SEPICIDE™ CI : | 0,50% |
| | Parfum : | 0,20% |
| | Ethanol à 95° : | 10,0% |

### MODE OPERATOIRE

Ajouter B dans A.

### Exemple 8 : Emulsion satinée pour le corps

**FORMULE**

| | | |
|---|---|---|
| A | SIMULSOL™ 165 : | 5,0% |
| | LANOL™ 1688 : | 8,50% |
| | Beurre de Karité : | 2% |
| | Huile de paraffine : | 6,5% |
| | LANOL™ 14 M : | 3% |
| | LANOL™ S : | 0,6% |
| B | Eau : | 66,2% |
| C | MICROPEARL™ M 100 : | 5% |
| D | Latex inverse auto-inversible (4) : | 3% |
| E | SEPICIDE™ Cl | 0,3% |
| | SEPICIDE™ HB : | 0,5% |
| | MONTEINE™ CA : | 1% |
| | Parfum : | 0,20% |
| | Acétate de vitamine E : | 0,20% |
| | pyrolidinonecarboxylate de sodium (agent hydratant) : | 1% |

### MODE OPERATOIRE

Ajouter C dans B, émulsionner B dans A à 70°C, puis ajouter D à 60°C puis E à 30°C.

### Exemple 9 : Lait corporel

**FORMULE**

| | | |
|---|---|---|
| A | SIMULSOL™ 165 : | 5,0% |
| | LANOL™ 1688 : | 12,0% |
| | LANOL™ 14 M : | 2,0% |
| | Alcool cétylique : | 0,3% |
| | SCHERCEMOL™ OP : | 3% |
| | B Eau: | q.s.p. 100% |
| C | Latex inverse auto-inversible (3) : | 0,35% |
| D | SEPICIDE™ CI : | 0,2% |
| | SEPICIDE™ HB : | 0,5% |
| | Parfum : | 0,20% |

### MODE OPERATOIRE

Emulsionner B dans A à 70°C, ajouter C, puis ajouter D à 60°C puis E à 30°C.

### Exemple 10 : crème H/E

**FORMULE**

| | | |
|---|---|---|
| A | SIMULSOL™ 165 : | 5,0% |
| | LANOL™ 1688 : | 20,0% |
| | LANOL™ P : | 1,0% |
| B | Eau : | q.s.p. 100% |
| C | Latex inverse auto-inversible (2) : | 2,50% |
| D | SEPICIDE™ CI : | 0,20% |
| | SEPICIDETM HB : | 0,30% |

### MODE OPERATOIRE

Introduire B dans A vers 75°C ; ajouter C vers 60°C, puis D vers 45°C

### Exemple 11 : gel solaire non gras

**FORMULE**

| | | |
|---|---|---|
| A | Latex inverse auto-inversible (1) : | 3.00% |
| | Eau: | 30% |
| B | SEPICIDE™ C : | 0,20% |
| | SEPICIDE™ HB : | 0,30% |
| | Parfum : | 0,10% |
| C | Colorant : | qs |
| | Eau : | 30% |
| D | MICROPEARL™ M 100 : | 3,00% |
| | Eau : | q.s.p. 100% |
| E | Huile de silicone : | 2,0% |
| | PARSOL™ MCX : | 5,00% |

### MODE OPERATOIRE

Introduire B dans A ; ajouter C puis D, puis E.

### Exemple 12 : Lait solaire

**FORMULE**

| | | |
|---|---|---|
| A | SEPIPERL™ N : | 3,0% |
| | Huile de sésame : | 5,0% |
| | PARSOL™ MCX : | 5,0% |
| | Carraghénane : | 0,10% |
| B | Eau: | q.s.p.100% |
| C | Latex inverse auto-inversible (3) : | 0,80% |
| D | Parfum : | q.s. |
| | Conservateur : | q.s. |

### MODE OPERATOIRE

Emulsionner B dans A à 75°C puis ajouter C vers 60°C, puis D vers 30°C et ajuster le pH si nécessaire

### Exemple 13 : Gel de massage

**FORMULE**

| | | |
|---|---|---|
| A | Latex inverse auto-inversible (2) : | 3,5% |
| | Eau : | 20,0% |
| B | Colorant: | 2 gouttes/100 g |
| | Eau : | q.s. |
| C | Alcool: | 10% |
| | Menthol : | 0,10% |
| D | Huile de silicone : | 5,0% |

### MODE OPERATOIRE

Ajouter B dans A ; puis ajouter au mélange, C puis D

### Exemple 14 : gel soin de massage

**FORMULE**

| | | |
|---|---|---|
| A | Latex inverse auto-inversible (3) : | 3,00% |
| | Eau : | 30% |
| B | SEPICIDE™ Cl: | 0,20% |
| | SEPICIDE™ HB : | 0,30% |
| | Parfum : | 0,05% |
| C | colorant : | q.s. |
| | Eau : | q.s.p. 100% |
| D | MICROPEARL™ SQL : | 5,0% |
| | LANOL™ 1688 : | 2% |

### MODE OPERATOIRE

Préparer A ; additionner B, puis C, puis D.

### Exemple 15 : Gel coup d'éclat

**FORMULE**

| | | |
|---|---|---|
| A | Latex inverse auto-inversible (4) : | 4% |
| | Eau : | 30% |
| B | ELASTINE HPM : | 5,0% |
| C | MICROPEARL™ M 100 : | 3% |
| | Eau : | 5% |
| D | SEPICIDE™ CI : | 0,2% |
| | SEPICIDE™ HB : | 0,3% |
| | Parfum : | 0,06% |
| | Pyrolidinonecarboxylate de sodium à 50% : | 1% |
| | Eau: | q.s.p. 100% |

### MODE OPERATOIRE

Préparer A ; additionner B, puis C, puis D.

### Exemple 16 : Lait corporel

**FORMULE**

| | | |
|---|---|---|
| A | SEPIPERL™ N : | 3,0% |
| | Triheptonate de glycérol : | 10,0% |
| B | Eau : | q.s.p. 100% |
| C | Latex inverse auto-inversible (1) : | 1,0% |
| D | Parfum : | q.s. |
| | Conservateur : | q.s. |

### MODE OPERATOIRE

Fondre A à environ 75°C. Emulsionner B dans A à 75°C puis ajouter C vers 60°C, puis D.

### Exemple 17 : Emulsion démaquillante à l'huile d'amandes douces

**FORMULE**

| | |
|---|---|
| MONTANOV™ 68 : | 5% |
| Huile d'amandes douces : | 5% |
| Eau : | q.s.p. 100% |
| Latex inverse auto-inversible (4) : | 0,3% |
| Glycérine : | 5% |
| Conservateur : | 0,2% |
| Parfum : | 0,3% |

### Exemple 18 : Crème hydratante pour peaux grasses

**FORMULE**

| | |
|---|---|
| MONTANOV™ 68 : | 5% |
| Octanoate de cétylstéaryle : ² | 8% |
| Palmitate d'octyle : | 2% |
| Eau: | q.s.p. 100% |
| Latex inverse auto-inversible (3) : | 0,6% |
| MICROPEARL™ M 100 : | 3,0% |
| Mucopolysaccharides : | 5% |
| SEPICIDE™ HB : | 0,8% |
| Parfum : | 0,3% |

### Exemple 19 : Baume après-rasage apaisant sans alcool

**FORMULE**

| | |
|---|---|
| Mélange de N-lauryl aminoacides : | 0,1% à 5% |
| Aspartate de magnésium et de potassium : | 0,002% à 0,5% |
| LANOL™ 99 : | 2% |
| Huile d'amandes douces : | 0,5% |
| Eau : | q.s.p. 100% |
| Latex inverse auto-inversible (2) : | 3% |
| SEPICIDE™ HB : | 0,3% |
| SEPICIDE™ Cl : | 0,2% |
| Parfum : | 0,4% |

### Exemple 20 : Crème aux AHA pour peaux sensibles

**FORMULE**

| | |
|---|---|
| Mélange de N-lauryl aminoacides : | 0,1% à 5% |
| Aspartate de magnésium et de potassium : | 0,002% à 0,5% |
| LANOL™ 99 : | 2% |
| MONTANOV™ 68 : | 5,0% |
| Eau : | q.s.p. 100% |
| Latex inverse auto-inversible (1) : | 1,50% |
| Acide gluconique : | 1,50% |
| Triéthanolamine : | 0,9% |
| SEPICIDE™ NB : | 0,3% |
| SEPICIDE™ Cl : | 0,2% |
| Parfum : | 0,4% |

### Exemple 21 : Soin apaisant après-soleil

**FORMULE**

| | |
|---|---|
| Mélange de N-lauryl aminoacides : | 0,1 % à 5% |
| Aspartate de magnésium et de potassium : | 0,002% à 0,5% |
| LANOL™ 99 : | 10,0% |
| Eau: | q.s.p. 100% |
| Latex inverse auto-inversible (4) : | 2,50% |
| SEPICIDE™ HB : | 0,3% |
| SEPICIDE™ CI : | 0,2% |
| Parfum : | 0,4% |
| Colorant : | 0,03% |

### Exemple 22 : Lait démaquillant

**FORMULE**

| | |
|---|---|
| SEPIPERL™ N : | 3% |
| PRIMOL™ 352 : | 8,0% |
| Huile d'amandes douces : | 2% |
| Eau: | q.s.p. 100% |
| Latex inverse auto-inversible (3) : | 0,8% |
| Conservateur : | 0,2% |

### Exemple 23 : Lait corporel

**FORMULE**

| | |
|---|---|
| SEPIPERL™ N : | 3,5% |
| LANOL™ 37T : | 8,0% |
| SOLAGUM™ L : | 0,05% |
| Eau : | q.s.p. 100% |
| Benzophénone : | 2,0% |
| Diméthicone 350 cPs : | 0,05% |
| Latex inverse auto-inversible (1) : | 0,8% |
| Conservateur : | 0,2% |
| Parfum : | 0,4% |

### Exemple 24 : émulsion fluide à pH alcalin

| | |
|---|---|
| MARCOL™ 82 : | 5,0% |
| NaOH | 10,0% |
| Eau : | q.s.p. 100% |
| Latex inverse auto-inversible (2) : | 1,5% |
| LANOL™ 84D : | 8,0% |
| LANOL™ 99 : | 5,0% |
| Eau : | q.s.p. 100% |
| Pigments et charges minérales : | 10,0% |
| Latex inverse auto-inversible (3) : | 1,2% |
| Conservateur : | 0,2% |
| Parfum : | 0,4% |

### Exemple 25 : Lait solaire

**FORMULE**

| | |
|---|---|
| SEPIPERL™ N : | 3,5% |
| LANOL™ 37T : | 10,0% |
| PARSOL™ NOX : | 5,0% |
| EUSOLEX™ 4360 : | 2,0% |
| Eau: | q.s.p. 100% |
| Latex inverse auto-inversible (4) : | 1,8% |
| Conservateur : | 0,2% |
| Parfum : | 0,4% |

### Exemple 26 : Gel contour des yeux

**FORMULE**

| | |
|---|---|
| Latex inverse auto-inversible (1): | 2,0% |
| Parfum : | 0,06% |
| pyrrolidinonecarboxylate de sodium : | 0,2% |
| DOW CORNING™ 245 FLuid : | 2,0% |
| Eau : | q.s.p. 100% |

### Exemple 27 : Composition de soin non rincée

**FORMULE**

| | |
|---|---|
| Latex inverse auto-inversible (2) : | 1,5% |
| Parfum : | q.s. |
| Conservateur : | q.s. |
| DOW CORNING™ X2 8360 : | 5,0% |
| DOW CORNING™ Q2 1401 : | 15,0% |
| Eau : | q.s.p. 100% |

### Exemple 28 : Gel amincissant

| | |
|---|---|
| Latex inverse auto-inversible (4) : | 5% |
| Ethanol: | 30% |
| Menthol : | 0,1% |
| Caféine : | 2,5% |
| Extrait de ruscus : | 2% |
| Extrait de lierre : | 2% |
| SEPICIDE™ HB : | 1% |
| Eau: | q.s.p. 100% |

### Exemple 29 : Baume après-rasage apaisant sans alcool

**FORMULE**

| | | |
|---|---|---|
| A | LIPACIDE™ PVB : | 1,0% |
| | LANOL™ 99 : | 2,0% |
| | Huile d'amandes douces : | 0,5% |
| B | Latex inverse auto-inversible (3) : | 3,5% |
| C | Eau : | q.s.p. 100% |
| D | Parfum : | 0,4% |
| | SEPICIDE™ HB : | 0,4% |
| | SEPICIDE™ CI : | 0,2% |

### Exemple 30 : Gel rafraîchissant après-rasage

**FORMULE**

| | | |
|---|---|---|
| | LIPACIDE™ PVB : | 0,5% |
| | LANOL™ 99 : | 5,0% |
| | Latex inverse auto-inversible (2) : | 2,5% |
| B | eau : | q.s.p. 100% |
| C | MICROPEARL™ LM : | 0,5% |
| | Parfum : | 0,2% |
| | SEPICIDE™ HB : | 0,3% |
| | SEPICIDE™ CI : | 0,2% |

### Exemple 31 : Soin pour les peaux grasses

**FORMULE**

| | | |
|---|---|---|
| A | MICROPEARL™ M310 : | 1,0% |
| | Latex inverse auto-inversible (4) : | 5.0% |
| | Isononanoate d'octyle : | 4,0% |
| B | Eau : q.s.p. | 100% |
| C | SEPICONTROL™ A5 : | 4,0% |
| | Parfum : | 0,1% |
| | SEPICIDE™ HB : | 0,3% |
| | SEPICIDE™ CI : | 0,2% |
| D | CAPIGEL™ 98 : | 0,5% |
| | Eau : | 10% |

### Exemple 32 : Crème aux AHA

**FORMULE**

| | | |
|---|---|---|
| A | MONTANOV™ 68 : | 5,0% |
| | LIPACIDE™ PVB : | 1,05% |
| | LANOL™ 99 : | 10,0% |
| B | Eau : | q.s.p. 100% |
| | Acide gluconique : | 1,5% |
| | Triéthanolamine : | 0,9% |
| C | Latex inverse auto-inversible (1) : | 1,5% |
| D | Parfum : | 0,4% |
| | SEPICIDE™ HB : | 0,2% |
| | SEPICIDE™ CI : | 0,4% |

### Exemple 33 : Autobronzant non gras pour visage et corps

**FORMULE**

| | | |
|---|---|---|
| A | LANOL™ 2681 : | 3,0% |
| | Latex inverse auto-inversible (4) | 2,5% |
| B | Eau : | q.s.p. 100% |
| | Dihydroxyacétone : | 3,0% |
| C | Parfum : | 0,2% |
| | SEPICIDE™ HB : | 0,8% |
| | Hydroxyde de sodium : | qs.pH = 5 |

### Exemple 34 : Lait solaire au monoï de Tahiti

**FORMULE**

| | | |
|---|---|---|
| A | Monoï de Tahiti : | 10% |
| | LIPACIDE™ PVB : | 0,5% |
| | Latex inverse auto-inversible (2) : | 2,2% |
| B | Eau: | q.s.p. 100% |
| C | Parfum : | 0,1% |
| | SEPICIDE™ HB : | 0,3% |
| | SEPICIDE™ Cl : | 0,1% |
| | Méthoxycinnamate d'octyle : | 4,0% |

### Exemple 35 : Soin solaire pour le visage

**FORMULE**

| | | |
|---|---|---|
| A | Cyclométhicone et diméthiconol : | 4,0% |
| | Latex inverse auto-inversible (3) : | 3,5% |
| B | Eau : | q.s.p. 100% |
| C | Parfum : | 0,1% |
| | SEPICIDE™ HB : | 0,3% |
| | SEPICIDE™ CI : | 0,21% |
| | Méthoxycinnamate d'octyle : | 5,0% |
| | Micatitane : | 2,0% |
| | Acide lactique : | q.s.p.pH 6,5 |

### Exemple 36 Emulsion bronzante sans soleil

**FORMULE**

| | | |
|---|---|---|
| A | LANOL™ 99: | 15% |
| | MONTANOV™ 68 : | 5,0% |
| | Paraméthoxycinnamate d'octyle : | 3,0% |
| B | Eau : | q.s.p. 100% |
| | Dihydroxyacétone : | 5,0% |
| | Phosphate monosodique : | 0,2% |
| C | Latex inverse auto-inversible (4) : | 0,5% |
| D | Parfum : | 0,3% |
| | SEPICIDE™ HB : | 0,8% |
| | Hydroxyde de sodium : | q.s. pH 7,5. |

### Exemple 37 : Gel brillance

| | |
|---|---|
| Latex inverse auto-inversible (1) : | 1,5% |
| Silicone volatile : | 25% |
| Monopropylèneglycol : | 25% |
| Eau déminéralisée : | 10% |
| Glycérine : | qsp.100% |

### Exemple 38 : Gel amincissant

| | |
|---|---|
| Latex inverse auto-inversible (2) : | 1,5% |
| Isononanoate d'isononyle : | 2% |
| Caféine : | 5% |
| Ethanol : | 40% |
| MICROPEARL™ LM : | 2% |
| Eau déminéralisée : | qsp. 100% |
| Conservateur parfum : | qs |

### Exemple 39 : Lait démaquillant

| | |
|---|---|
| SIMULSOL™ 165 : | 4% |
| MONTANOV™ 202 : | 1% |
| Caprylate-caprate triglyceride : | 15% |
| PECOSIL™ DCT : | 1% |
| Eau déminéralisée: | qsp. 100% |
| CAPIGEL™ 98 : | 0,5% |
| Latex inverse auto-inversible (3) : | 1% |
| PROTEOL™ OAT : | 2% |
| Hydroxyde de sodium : | qsp pH = 7 |

### Exemple 40 : Masque crème "rince off" restructurant pour cheveux stressés et fragilisés

**FORMULE**

| | |
|---|---|
| KETROL™T : | 0,5% |
| PECOSIL™ SPP50 : | 0,75% |
| N-cocoyl aminoacides : | 0,70% |
| Butylèneglycol : | 3,0% |
| Latex inverse auto-inversible (1) : | 3,0% |
| MONTANOV™ 82 : | 3,0% |
| Huile de jojoba : | 1,0% |
| LANOL™ P : | 6,0% |
| AMONYL™ DM : | 1,0% |
| LANOL™99 : | 5,0% |
| SEPICIDE™ HB | 0,3% |
| SEPICIDE™CI : | 0,2% |
| Parfum : | 0,2% |
| Eau : qsp | 100% |

### Exemple 41 : Crème solaire

| | |
|---|---|
| SIMULSOL™ 165 : | 3% |
| MONTANOV™ 202: | 2% |
| Benzoate C12-C15 : | 8% |
| PECOSIL™ PS 100 : | 2% |
| Diméthicone : | 2% |
| Cyclométhicone : | 5% |
| para-méthoxy cinnamate d'octyle : | 6% |
| Benzophénone-3 : | 4% |
| Oxyde de Titane : | 8% |
| Gomme xanthane : | 0,2% |
| Butylèneglycol : | 5% |
| Eau déminéralisée: | qsp. 100% |
| Latex inverse auto-inversible (2) : | 1,5% |
| Conservateur, parfum : | qs |

### Exemple 42 : Gel de soin peaux mixtes

| | |
|---|---|
| Latex inverse auto-inversible (3) : | 4% |
| Squalane végétal : | 5% |
| Dimethicone : | 1,5% |
| SEPICONTROL™ A5 : | 4% |
| Gomme xanthane : | 0,3% |
| Eau: | qsp.100% |
| Conservateur, Parfum : | qs. |

### Exemple 43 : Lotion capillaire

**FORMULE**

| | |
|---|---|
| Butylène glycol : | 3,0% |
| Latex inverse auto-inversible (4) : | 3% |
| SIMULSOL™1293 : | 3,0% |
| Acide lactique : | qs. pH = 6 |
| SEPICIDE™ HB : | 0,2% |
| SEPICIDE™C1 : | 0,3% |
| Parfum : | 0,3% |
| Eau : | qsP. 100% |

### Exemple 44 : Shampooing protecteur et relaxant

**FORMULE**

| | |
|---|---|
| Amonyl™ 675 SB : | 5,0% |
| Sodium lauryl éther sulfate à 28% : | 35,0% |
| Composition de l'exemple 1 : | 3,0% |
| SEPICIDE™ HB : | 0,5% |
| SEPICIDE™CI : | 0,3% |
| Hydroxyde de sodium : | QS pH = 7,2 |
| Parfum : | 0,3% |
| Colorant (FDC bleu 1/jaune 5) : | QS |
| Eau: QSP 100% | |

### Exemple 45 : Protecteur "leave-on" ; Soin Formule anti-stress pour cheveux

**FORMULE**

| | |
|---|---|
| KETROL™T : | 0,5% |
| mélange de cocoyl aminoacides : | 3,0% |
| Butylèneglycol : | 5,0% |
| DC 1501: | 5,0% |
| composition de l'exemple 2 : | 4,0% |
| SEPICIDE™ HB : | 0,5% |
| SEPIC1DE™CI : | 0,3% |
| Parfum : | 0,3% |
| Eau: | QSP 100% |

### Exemple 46 : Crème vitaminée

| | |
|---|---|
| SIMULSOL™ 165 : | 5% |
| MONTANOV™ 202 : | 1% |
| Caprylic/capric triglycérides : | 20% |
| Palmitate de vitamine A : | 0,2% |
| Acétate de vitamine E : | 1% |
| MICROPEARL™ M 305: | 1,5% |
| Composition de l'exemple 3 : | 2% |
| Eau : | qsp 100% |
| Conservateur, parfum : | qs |

Les définitions des produits commerciaux utilisés dans les exemples sont les suivantes : SIMULSOL™ 1293 est de l'huile de castor hydrogénée et polyéthoxylée, avec un indice d'éthoxylation égal à 40, commercialisé par la société SEPPIC.
CAPIGEL™ 98 est un épaississant liquide à base de copolymère acrylate commercialisé par la société SEPPIC.
KETROL™T est de la gomme de xanthane commercialisée par la société KELCO. LANOL™ 99 est de l'isononyl isononanoate commercialisé par la société SEPPIC.
DC1501 est un mélange de cyclopentasiloxane et de diméthiconol commercialisé par la société DOW CHEMICAL.
MONTANOV™ 82 est un agent émulsionnant à base d'alcool cétéarylique et de cocoylglucos ide.
Le MONTANOV™ 68 (cétéaryl glucoside), est une composition auto-émulsionnable telle que décrite dans WO 92/06778, commercialisée par la société SEPPIC.
Le MICROPEARL™ M 100 est une poudre ultra fine au toucher très doux et à action matifiante commercialisée par la société MATSUMO.
Le SEPICIDE™ CI, imidazolidine urée, est un agent conservateur commercialisé par la société SEPPIC.
PEMULEN™ TR est un polymère acrylique commercialisé par GOODRICH.
Le SIMULSOL™165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL S sont des facteurs de consistance commercialisés par la société SEPPIC.
Le SEPICIDE™ HB, qui est un mélange de phénoxy éthanol, de méthyl paraben, d'éthyl paraben, de propyl paraben et de butyl paraben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le PARSOL™ MCX est du (para-méthoxy) cinnamate d'octyle; commercialisé par la société GIVAUDAN.
Le SEPIPERL™ N est un agent nacrant, commercialisé par la société SEPPIC, à base d'un mélange d'alkyl polyglucosides tels que ceux décrits dans WO 95/13863.
Le MICROPEARL™ SQL est un mélange de micro particules renfermant du squalane qui se libère sous l'action du massage; il est commercialisé par la société MATSUMO.
Le LANOL™ 99 est de l'isononanoate d'isononyle commercialisé par la société SEPPIC.
Le LANOL™ 37T est du triheptanoate de glycérol, commercialisé par la société SEPPIC.
Le SOLAGUM™ L est un carraghénane commercialisé par la société SEPPIC.
Le MARCOL™ 82 est une huile de paraffine commercialisée par la société EXXON. Le LA-NOL™ 84D est du malate de dioctyle commercialisé par la société SEPPIC. Le PARSOL NOX ™ est un filtre solaire commercialisé par la société GIVAUDAN.
EUSOLEX™ 4360 est un filtre solaire commercialisé par la société MERCK.
Le DOW CORNING™ 245 Fluid est de la cyclométhicone, commercialisée par la société DOW CORNING.
Le LIPACIDE™ PVB, est un hydrolysat de protéines de blé acyle commercialisé par la société SEPPIC.
Le MICROPEARL™ LM est un mélange de squalane, de polyméthylméthacrylate et de menthol, commercialisé par la société SEPPIC.
Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
Le LANOL™ 2681 est un mélange caprylate, caprate de coprah, commercialisé par la société SEPPIC.
Le MONTANOV™ 202, est une composition APG/alcools gras telle que décrite dans WO9 98/47610, commercialisée par la société SEPPIC.

## Revendications

1. Composition sous forme d'un latex inverse auto-inversible, comprenant pour 100% de sa masse :
a) - De 50% massique à 70% massique d'un polyélectrolyte anionique (P) réticulé, obtenu par polymérisation :
- D'au moins un monomère neutre de formule (I) : 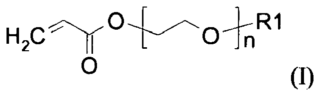 dans laquelle le radical R1 représente un radical aliphatique linéaire ou ramifié, comportant de 8 à 20 atomes de carbone et n représente un nombre supérieur ou égal à un et inférieur ou égal à trente ;
- D'au moins un monomère neutre choisi parmi l'acrylamide, le N,N-diméthyl acrylamide, le N-[2-hydroxy-1,1-bis(hydroxyméthyl) éthyl] propènamide [ou tris(hydroxyméthyl) acrylamidométhane ou N-[tris(hydroxyméthyl) méthyl] acrylamide dénommé aussi THAM] ou l'acrylate de (2-hydroxy éthyle) ; et
- D'au moins un monomère comportant une fonction acide fort et/ou d'au moins un monomère comportant une fonction acide faible ;
**b) -** De 4% massique à 10% massique d'un système émulsionnant (S₁) de type eau dans huile (E/H) ;
**c) -** De 1% massique à 10% massique d'un système émulsionnant (S₂) de type huile dans eau (H/E) comprenant une proportion massique non nulle d'une composition tensioactive (C), ladite composition tensioactive (C) comprenant pour 100% molaire :
**1) -** Une proportion supérieure ou égale à 10% molaire et inférieure ou égale à 50% molaire d'une composition (C_{II}) comprenant pour 100% molaire :
**α)** - De 60% molaire à 100% molaire d'un composé de formule (II) : 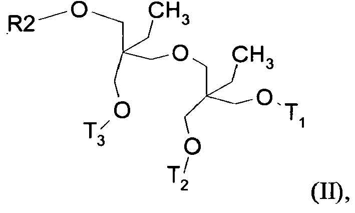 dans laquelle :
- R2 représente un radical alkyle linéaire ou ramifié, comportant de 12 atomes de carbone,
- T₁, représente un atome d'hydrogène ou un radical (-CH₂-CH₂-O-)ₘ₁-H dans lequel m1 est un nombre entier supérieur ou égal à zéro et inférieur ou égal à dix,
- T₂ identique ou différent de T₁, représente un atome d'hydrogène ou un radical (-CH₂-CH₂-O-)ₘ₂-H dans lequel m2 est un nombre entier supérieur ou égal à zéro et inférieur ou égal à dix, et
- T₃ identique ou différent de T₁ et de T₂, représente un atome d'hydrogène ou un radical (-CH₂-CH₂-O-)ₘ₃-H dans lequel m3 est un nombre entier supérieur ou égal à zéro et inférieur ou égal à dix,
- étant entendu que la somme m1 + m2 + m3 est supérieure à 0 et inférieure ou égale à dix ;
**β**) - Optionnellement jusqu'à 40% molaire d'un composé de formule (II') : 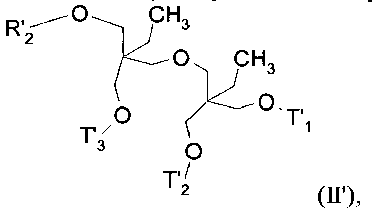 dans laquelle :
- R'₂ représente un radical alkyle linéaire ou ramifié, comportant 14 atomes de carbone,
- T'₁, représente un atome d'hydrogène ou un radical (-CH₂-CH₂-O-)ₘ₁-H dans lequel m1 est un nombre entier supérieur ou égal à zéro et inférieur ou égal à dix,
- T'₂ identique ou différent de T'₁, représente un atome d'hydrogène ou un radical (-CH₂-CH₂-O-)ₘ₂-H dans lequel m2 est un nombre entier supérieur ou égal à zéro et inférieur ou égal à dix, et
- T'₃ identique ou différent de T'₁ et de T'₂, représente un atome d'hydrogène ou un radical (-CH₂-CH₂-O-)ₘ₃-H dans lequel m3 est un nombre entier supérieur ou égal à zéro et inférieur ou égal à dix,
- étant entendu, que la somme m1 + m2 + m3 est supérieure à 0 et inférieure ou égale à dix ; et
**γ)** - Optionnellement jusqu'à 10% molaire d'un composé de formule (II") : 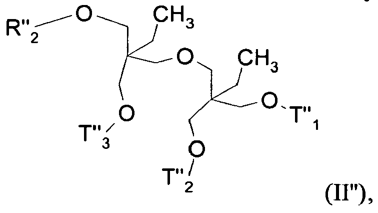 dans laquelle :
- R"₂ représente un radical alkyle linéaire ou ramifié, comportant 16 atomes de carbone,
- T"₁, représente un atome d'hydrogène ou un radical (-CH₂-CH₂-O-)ₘ₁-H dans lequel m1 est un nombre entier supérieur ou égal à zéro et inférieur ou égal à dix,
- T"₂ identique ou différent de T"₁, représente un atome d'hydrogène ou un radical (-CH₂-CH₂-O-)ₘ₂-H dans lequel m2 est un nombre entier supérieur ou égal à zéro et inférieur ou égal à dix, et
- T"₃ identique ou différent de T"₁ et de T"₂, représente un atome d'hydrogène ou un radical (-CH₂-CH₂-O-)ₘ₃-H dans lequel m3 est un nombre entier supérieur ou égal à zéro et inférieur ou égal à dix,
- étant entendu que la somme m1 + m2 + m3 est supérieure à 0 et inférieure ou égale à dix ;
**2) -** Une proportion supérieure ou égale à 50% molaire et inférieure ou égale à 90% molaire d'une composition (C_{III}) comprenant pour 100% molaire :
**α)** - De 60% molaire à 100% molaire d'un composé de formule (III) : 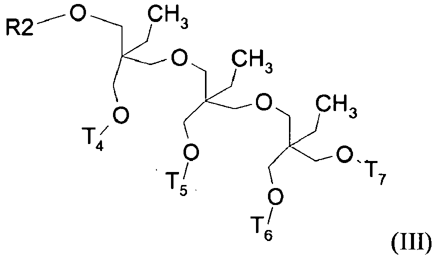 ou de son isomère de formule (IV) : 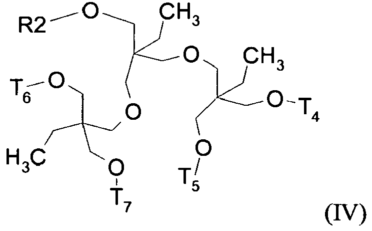 ou du mélange de ces deux isomères,
formules (III) et (IV) dans lesquelles :
- R2 représente un radical alkyle linéaire ou ramifié, comportant 12 atomes de carbone,
- T₄, représente un atome d'hydrogène ou un radical (-CH₂-CH₂-O-)ₘ₄-H dans lequel m4 est un nombre entier supérieur ou égal à zéro et inférieur ou égal à dix,
- T₅ identique ou différent de T₄, représente un atome d'hydrogène ou un radical (-CH₂-CH₂-O-)ₘ₅-H dans lequel m5 est un nombre entier supérieur ou égal à zéro et inférieur ou égal à dix,
- T₆ identique ou différent de T₄ et de T₅ représente un atome d'hydrogène ou un radical (-CH₂-CH₂-O-)ₘ₆-H dans lequel m6 est un nombre entier supérieur ou égal à zéro et inférieur ou égal à dix,
- T₇ identique ou différent de T₄ de T₅ et de T₆, représente un atome d'hydrogène ou un radical (-CH₂-CH₂-O-)ₘ₇-H dans lequel m7 est un nombre entier supérieur ou égal à zéro et inférieur ou égal à dix étant entendu que la somme m4 + m5 + m6 + m7 est supérieure à 0 et inférieure ou égale à dix ;
**P) -** Optionnellement jusqu'à 40% molaire composé de formule (III') : 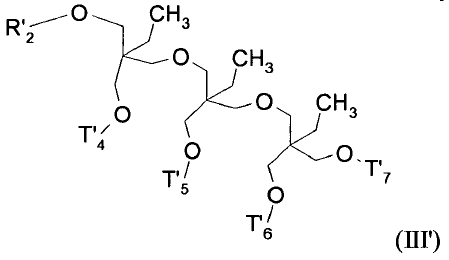 ou de son isomère de formule (IV') : 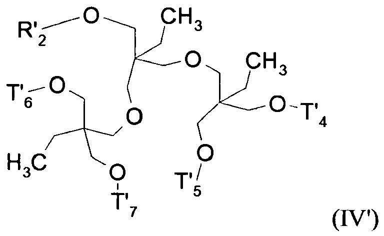 ou du mélange de ces deux isomères,
formules (III') et (IV') dans lesquelles :
- R'₂ représente un radical alkyle linéaire ou ramifié, comportant 14 atomes de carbone,
- T'₄, représente un atome d'hydrogène ou un radical (-CH₂-CH₂-O-)ₘ₄-H dans lequel m4 est un nombre entier supérieur ou égal à zéro et inférieur ou égal à dix,
- T'₅ identique ou différent de T'₄, représente un atome d'hydrogène ou un radical (-CH₂-CH₂-O-)ₘ₅-H dans lequel m5 est un nombre entier supérieur ou égal à zéro et inférieur ou égal à dix,
- T'₆ identique ou différent de T'₄ et de T'₅, représente un atome d'hydrogène ou un radical (-CH₂-CH₂-O-)ₘ₆-H dans lequel m6 est un nombre entier supérieur ou égal à zéro et inférieur ou égal à dix, et
- T'₇ identique ou différent de T'₄, de T'₅ et de T'₆, représente un atome d'hydrogène ou un radical (-CH₂-CH₂-O-)ₘ₇-H dans lequel m7 est un nombre entier supérieur ou égal à zéro et inférieur ou égal à dix,
- étant entendu que la somme m4 + m5 + m6 + m7 est supérieure à 0 et inférieure ou égale à dix ; et
**γ)** - Optionnellement jusqu'à 10% molaire d'un composé de formule (III") : 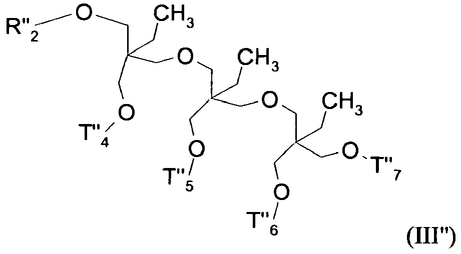
ou de son isomère de formule (IV") : 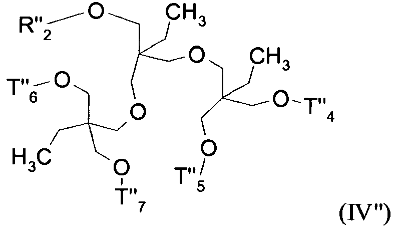
ou du mélange de ces deux isomères,
formules (III") et (IV") dans lesquelles :
- R"₂ représente un radical alkyle linéaire ou ramifié, comportant 16 atomes de carbone,
- T"₄, représente un atome d'hydrogène ou un radical (-CH₂-CH₂-O-)ₘ₄-H dans lequel m4 est un nombre entier supérieur ou égal à zéro et inférieur ou égal à dix,
- T"₅ identique ou différent de T"₄, représente un atome d'hydrogène ou un radical (-CH₂-CH₂-O-)ₘ₅-H dans lequel m5 est un nombre entier supérieur ou égal à zéro et inférieur ou égal à dix,
- T"₆ identique ou différent de T"₄ et de T"₅ représente un atome d'hydrogène ou un radical (-CH₂-CH₂-O-)ₘ₆-H dans lequel m6 est un nombre entier supérieur ou égal à zéro et inférieur ou égal à dix, et
- T"₇ identique ou différent de T"₄, de T"₅ et de T"₆, représente un atome d'hydrogène ou un radical (-CH₂-CH₂-O-)ₘ₇-H dans lequel m7 est un nombre entier supérieur ou égal à zéro et inférieur ou égal à dix,
- étant entendu que la somme m4 + m5 + m6 + m7 est supérieure à 0 et inférieure ou égale à dix ;
**d) -** De 15% massique à 45% massique d'au moins une huile, et
**e) -** De 0% massique à 5% massique d'eau.

2. Composition telle que définie à la revendication 1, pour laquelle dans la formule (I), le radical R1 représente un radical lauryle ou un radical stéaryle.

3. Composition telle que définie à l'une des revendications 1 ou 2, pour laquelle dans la formule (I), n est supérieur ou égal à deux et inférieur ou égal à vingt.

4. Composition telle que définie à l'une quelconque des revendications 1 à 3, pour laquelle dans le polyélectrolyte P, les unités monomériques comportant une fonction acide fort sont issues de l'acide 2-méthyl 2-[(1-oxo 2-propènyl) amino] 1-propanesulfonique partiellement ou totalement salifié en sel de sodium, en sel de potassium, ou en sel d'ammonium et les unités monomériques comportant une fonction acide faible sont issues de l'acide acrylique ou de l'acide méthacrylique, partiellement salifié en sel de sodium, en sel de potassium, ou en sel d'ammonium.

5. Composition telle que définie à l'une quelconque des revendications 1 à 4, pour laquelle le polyélectrolyte P comprend, en pourcentage molaire, de 0,5% à 10% d'une unité monomérique issue du monomère de formule (I) telle que définie précédemment.

6. Composition telle que définie à l'une quelconque des revendications 1 à 5, pour laquelle ladite composition tensioactive (C) comprend en outre :
**3) -** Jusqu'à 5% molaire d'une composition (C_{V}) comprenant pour 100% molaire :
**α)** - De 60% molaire à 100% molaire d'un composé de formule (V) : 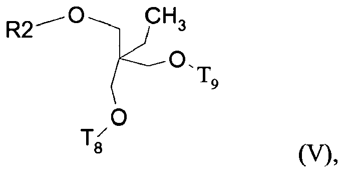 dans laquelle :
- R2 représente un radical alkyle linéaire ou ramifié, comportant de 12 atomes de carbone,
- T₈, représente un atome d'hydrogène ou un radical (-CH₂-CH₂-O-)ₘ₈-H dans lequel m8 est un nombre entier supérieur ou égal à zéro et inférieur ou égal à dix,
- T₉ identique ou différent de T₈, représente un atome d'hydrogène ou un radical (-CH₂-CH₂-O-)ₘ₉-H dans lequel m9 est un nombre entier supérieur ou égal à zéro et inférieur ou égal à dix, et
- étant entendu que la somme m8 + m9 est supérieure à 0 et inférieure ou égale à dix ;
**β)** - Optionnellement jusqu'à 40% molaire d'un composé de formule (V') : 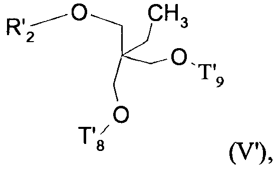 dans laquelle :
- R'₂ représente un radical alkyle linéaire ou ramifié, comportant 14 atomes de carbone,
- T'₈, représente un atome d'hydrogène ou un radical (-CH₂-CH₂-O-)ₘ₈-H dans lequel m8 est un nombre entier supérieur ou égal à zéro et inférieur ou égal à dix,
- T'₉ identique ou différent de T'₈, représente un atome d'hydrogène ou un radical (-CH₂-CH₂-O-)ₘ₉-H dans lequel m9 est un nombre entier supérieur ou égal à zéro et inférieur ou égal à dix, et
- étant entendu que la somme m8 + m9 est supérieure à 0 et inférieure ou égale à dix ; et
**γ)** - Optionnellement jusqu'à 10% molaire d'un composé de formule (V") : 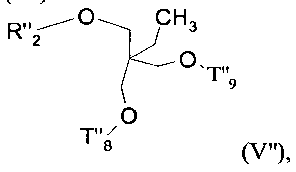 dans laquelle :
- R"₂ représente un radical alkyle linéaire ou ramifié, comportant 16 atomes de carbone,
- T"₈, représente un atome d'hydrogène ou un radical (-CH₂-CH₂-O-)ₘ₈-H dans lequel m8 est un nombre entier supérieur ou égal à zéro et inférieur ou égal à dix,
- T"₉ identique ou différent de T"₈, représente un atome d'hydrogène ou un radical (-CH₂-CH₂-O-)ₘ₉-H dans lequel m9 est un nombre entier supérieur ou égal à zéro et inférieur ou égal à dix, et
- étant entendu que la somme m8 + m9 est supérieure à 0 et inférieure ou égale à dix.

7. Composition telle que définie à l'une quelconque des revendications 1 à 6, pour laquelle ladite composition tensioactive (C) comprend en outre :
**4) -** Jusqu'à 5% molaire d'une composition (C_{VI}) comprenant pour 100% molaire :
**α)** - De 60% molaire à 100% molaire d'un composé de formule (VI) :
R-2-(-CH₂-CH₂-O-)ₘ₁₀-H (VI),
dans laquelle R2 représente un radical alkyle linéaire ou ramifié, comportant 12 atomes de carbone et m10 est un nombre entier supérieur ou égal à zéro et inférieur ou égal à dix ;
**β)** - Optionnellement jusqu'à 40% molaire d'un composé de formule (VI') :
R'₂-(-CH₂-CH₂-O-)ₘ₁₀-H (VI'),
dans laquelle R'₂ représente un radical alkyle linéaire ou ramifié, comportant 14 atomes de carbone et m10 est un nombre entier supérieur ou égal à zéro et inférieur ou égal à dix ; et
**γ)** - Optionnellement jusqu'à 10% molaire d'un composé de formule (VI") :
R"₂-(-CH₂-CH₂-O-)ₘ₁₀-H (VI"),
dans laquelle R"₂ représente un radical alkyle linéaire ou ramifié, comportant 16 atomes de carbone et m10 est un nombre entier supérieur ou égal à zéro et inférieur ou égal à dix.

8. Composition telle que définie à l'une quelconque des revendications 1 à 7, pour laquelle ladite composition tensioactive (C) comprend
**1) -** Une proportion supérieure ou égale à 20% molaire et inférieure ou égale à 50% molaire d'une composition (C_{II}) telle que définie précédemment ;
**2) -** Une proportion supérieure ou égale à 50% molaire et inférieure ou égale à 80% molaire d'une composition (C_{III}) telle que définie précédemment.

9. Composition telle que définie à l'une quelconque des revendications 1 à 8, pour laquelle, dans ladite composition tensioactive (C) :
**1) -** ladite composition (C_{II}) comprend pour 100% molaire :
**α**) - De 60% à 80% molaire du composé de formule (II),
**P) -** De 15% à 30% molaire du composé de formule (II'), et
**y) -** Jusqu'à 10% molaire du composé de formule (II"), et
**2) -** ladite composition (C_{III}) comprend pour 100% molaire :
**α**) - De 60% à 80% molaire du composé de formule (III), de son isomère de formule (IV) ou du mélange de ces isomères,
**P) -** de 15% à 30% molaire du composé de formule (III'), de son isomère de formule (IV') ou du mélange de ces isomères, et
**γ) -** Jusqu'à 10% molaire du composé de formule (III"), de son isomère de formule (IV") ou du mélange de ces isomères.

10. Composition telle que définie à l'une quelconque des revendications 1 à 9, dans laquelle ledit système émulsionnant (S₂) de type huile dans eau (H/E) consiste en 100% massique de ladite composition tensioactive (C).

11. Composition telle que définie à l'une quelconque des revendications 1 à 10, dans laquelle ledit système émulsionnant (S₂) de type huile dans eau (H/E) comprend pour 100% de sa masse :
- de 10% massique à 40% massique d'alcool laurique heptaéthoxylé et
- de 60% massique à 90% massique de ladite composition tensioactive (C).

12. Composition telle que définie à l'une quelconque des revendications 1 à 11, pour laquelle dans le polyélectrolyte (P), l'unité monomérique issue du monomère de formule (I) est une unité monomérique issus de l'acrylate de lauryle tétraéthoxylé.

13. Composition telle que définie à la revendication 12, dans laquelle le polyélectrolyte (P) est choisi parmi :
- Les copolymères réticulés de l'acide acrylique partiellement salifié sous forme de sel de sodium ou de sel d'ammonium, de l'acrylamide et de l'acrylate de lauryle tétraéthoxylé ;
- Les copolymères réticulés de l'acide 2-méthyl 2-[(1-oxo 2-propènyl) amino] 1-propanesulfonique partiellement salifié sous forme de sel de sodium ou de sel d'ammonium, de l'acrylamide et de l'acrylate de lauryle tétraéthoxylé ;
- Les copolymères réticulés de l'acide 2-méthyl 2-[(1-oxo 2-propènyl) amino] 1-propanesulfonique partiellement salifié sous forme de sel de sodium ou de sel d'ammonium, de l'acrylate de 2-hydroxy éthyle et de l'acrylate de lauryle tétraéthoxylé ;
- Les copolymères réticulés de l'acrylamide, de l'acide 2-méthyl 2-[(1-oxo 2-propènyl) amino] 1-propanesulfonique, de l'acide acrylique partiellement salifiés sous forme de sel sodium ou de sel d'ammonium et de l'acrylate de lauryle tétraéthoxylé ;
- Les copolymères de l'acide 2-méthyl 2-[(1-oxo 2-propènyl) amino] 1-propanesulfonique partiellement salifié sous forme de sel de sodium ou de sel d'ammonium, de l'acrylamide, du vinyl pyrrolidone et de l'acrylate de lauryle tétraéthoxylé ; et
- Les copolymères réticulés de l'acide 2-méthyl 2-[(1-oxo 2-propènyl) amino] 1-propanesulfonique partiellement ou totalement salifié sous forme de sel de sodium, de l'acide acrylique partiellement salifiés sous forme de sel sodium ou de sel d'ammonium, de l'acrylate de 2-hydroxy éthyle, de tris(hydroxyméthyl)aminométhyl acrylamide et de l'acrylate de lauryle tétraéthoxylé.

14. Composition telle que définie à l'une quelconque des revendications 1 à 13, dans laquelle le polyélectrolyte anionique (P) réticulé comporte pour 100% de monomères mis en oeuvre :
- De 40% molaire à 80% molaire d'unités monomériques issues d'un monomère comportant une fonction acide fort ;
- De 15% molaire à 55% molaire d'unités monomériques issues d'un monomère neutre différent du composé de formule (I) telle que définie précédemment ;
- De 1% à 5% molaire d'unités monomériques issues d'un monomère de formule (I) telle que définie précédemment.

15. Utilisation de la composition telle que définie à l'une quelconque des revendications 1 à 14, comme agent épaississant et/ou émulsionnant, pour préparer une composition topique cosmétique, dermopharmaceutique ou pharmaceutique.

16. Composition topique cosmétique, dermopharmaceutique ou pharmaceutique, **caractérisée en ce qu'**elle comprend, comme agent épaississant et/ou émulsionnant, une quantité efficace de la composition telle que définie à l'une quelconque des revendications 1 à 14.

## Patentansprüche

1. Zusammensetzung in Form eines inversen, auto-inversiblen Latex, die pro 100 % ihrer Masse Folgendes enthält:
a) 50 Masse-% bis 70 Masse-% eines vernetzten anionischen Polyelektrolyten (P), der durch Polymerisation erhalten wird,
- mindestens ein neutrales Monomer der Formel (I), 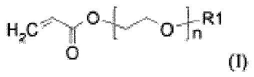 worin das Radikal R1 ein geradkettiges oder verzweigtes aliphatisches Radikal mit 8 bis 20 Kohlenstoffatomen darstellt und n eine Zahl ist, die größer oder gleich eins und kleiner oder gleich dreißig ist;
- mindestens ein neutrales Monomer, das ausgewählt ist aus Acrylamid, N,N-Dimethylacrylamid, N-[2-hydroxy-1,1-bis(hydroxymethyl)ethyl]propenamid [oder tris(hydroxymethyl)acrylamidomethan oder N-tris(hydroxymethyl)methyl]acrylamid, auch als THAM bezeichnet] oder (2-Hydroxyethyl)acrylat; und
- mindestens ein Monomer, das eine starke Säurefunktion aufweist, und/oder mindestens ein Monomer, das eine schwache Säurefunktion aufweist;
b) 4 Masse-% bis 10 Masse-% eines Emulgatorsystems (S₁) des Typs Wasser-in-Öl (W/O);
c) 1 Masse-% bis 10 Masse-% eines Emulgatorsystems (S₂) des Typs Öl-in-Wasser (O/W), das einen Masseanteil ungleich null einer Tensidzusammensetzung (C) enthält, wobei die Tensidzusammensetzung (C) für 100 Mol-% Folgendes enthält:
1) einen Anteil von größer oder gleich 10 Mol-% und kleiner oder gleich 50 Mol-% einer Zusammensetzung (C_{II}), enthaltend für 100 Mol-%:
α) 60 Mol-% bis 100 Mol-% einer Verbindung der Formel (II): 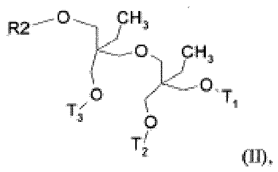 worin:
- R2 ein geradkettiges oder verzweigtes Alkylradikal mit 12 Kohlenstoffatomen darstellt,
- T₁ ein Wasserstoffatom oder ein (-CH₂-CH₂-O-)ₘ₁-H-Radikal darstellt, wobei m1 eine ganze Zahl ist, die größer oder gleich null und kleiner oder gleich zehn ist,
- T₂, das mit T₁ identisch oder von ihm verschieden ist, ein Wasserstoffatom oder ein (-CH₂-CH₂-O-)ₘ₂-H-Radikal darstellt, wobei m2 eine ganze Zahl ist, die größer oder gleich null und kleiner oder gleich zehn ist, und
- T₃, das mit T₁ und T₂ identisch oder von ihnen verschieden ist, ein Wasserstoffatom oder ein (-CH₂-CH₂-O-)ₘ₃-H-Radikal darstellt, wobei m3 eine ganze Zahl ist, die größer oder gleich null und kleiner oder gleich zehn ist,
- wobei die Summe aus m1 + m2 + m3 größer als null und kleiner oder gleich zehn ist;
β) optional bis zu 40 Mol-% einer Verbindung der Formel (II'): 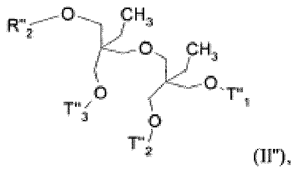 worin:
- R'₂ ein geradkettiges oder verzweigtes Alkylradikal mit 14 Kohlenstoffatomen darstellt,
- T'₁ ein Wasserstoffatom oder ein (-CH2-CH₂-O-)ₘ₁-H-Radikal darstellt, wobei m1 eine ganze Zahl ist, die größer oder gleich null und kleiner oder gleich zehn ist,
- T'₂, das mit T'₁ identisch oder von ihm verschieden ist, ein Wasserstoffatom oder ein (-CH₂-CH₂-O-)ₘ₂-H-Radikal darstellt, wobei m2 eine ganze Zahl ist, die größer oder gleich null und kleiner oder gleich zehn ist, und
- T'₃, das mit T'₁ und T'₂ identisch oder von ihnen verschieden ist, ein Wasserstoffatom oder ein (-CH₂-CH₂-O-)ₘ₃-H-Radikal darstellt, wobei m3 eine ganze Zahl ist, die größer oder gleich null und kleiner oder gleich zehn ist,
- wobei die Summe aus m1 + m2 + m3 größer als null und kleiner oder gleich zehn ist; und
γ) optional bis zu 10 Mol-% einer Verbindung der Formel (II"): 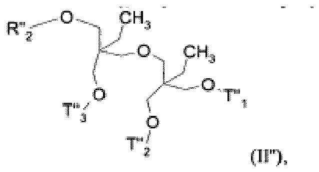 worin:
- R"₂ ein geradkettiges oder verzweigtes Alkylradikal mit 16 Kohlenstoffatomen darstellt,
- T"₁ ein Wasserstoffatom oder ein (-CH₂-CH₂-O-)ₘ₁-H-Radikal darstellt, wobei m1 eine ganze Zahl ist, die größer oder gleich null und kleiner oder gleich zehn ist,
- T"₂, das mit T"₁ identisch oder von ihm verschieden ist, ein Wasserstoffatom oder ein (-CH₂-CH₂-O-)ₘ₂-H-Radikal darstellt, wobei m2 eine ganze Zahl ist, die größer oder gleich null und kleiner oder gleich zehn ist, und
- T"₃, das mit T"₁ und T"₂ identisch oder von ihnen verschieden ist, ein Wasserstoffatom oder ein (-CH₂-CH₂-O-)ₘ₃-H-Radikal darstellt, wobei m3 eine ganze Zahl ist, die größer oder gleich null und kleiner oder gleich zehn ist,
- wobei die Summe aus m1 + m2 + m3 größer als null und kleiner oder gleich zehn ist;
2) einen Anteil von größer oder gleich 50 Mol-% und kleiner oder gleich 90 Mol-% einer Zusammensetzung (C_{III}), enthaltend für 100 Mol-%:
α) 60 Mol-% bis 100 Mol-% einer Verbindung der Formel (III): 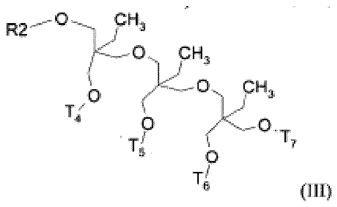 oder von deren Isomer der Formel (IV): 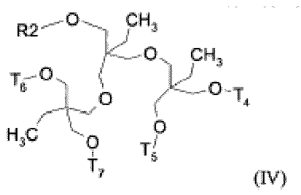 oder von dem Gemisch aus diesen zwei Isomeren, wobei in den Formeln (III) und (IV):
- R2 ein geradkettiges oder verzweigtes Alkylradikal mit 12 Kohlenstoffatomen darstellt,
- T₄ ein Wasserstoffatom oder ein (-CH₂-CH₂-O-)ₘ₄-H-Radikal darstellt, wobei m4 eine ganze Zahl ist, die größer oder gleich null und kleiner oder gleich zehn ist,
- T₅, das mit T₄ identisch oder von ihm verschieden ist, ein Wasserstoffatom oder ein (-CH₂-CH₂-O-)ₘ₅-H-Radikal darstellt, wobei m5 eine ganze Zahl ist, die größer oder gleich null und kleiner oder gleich zehn ist,
- T₆, das mit T₄ und T₅ identisch oder von ihnen verschieden ist, ein Wasserstoffatom oder ein (-CH₂-CH₂-O-)ₘ₆-H-Radikal darstellt, wobei m6 eine ganze Zahl ist, die größer oder gleich null und kleiner oder gleich zehn ist,
- T₇, das mit T₄, T₅ und T₆ identisch oder von ihnen verschieden ist, ein Wasserstoffatom oder ein (-CH₂-CH₂-O-)ₘ₇-H-Radikal darstellt, wobei m7 eine ganze Zahl ist, die größer oder gleich null und kleiner oder gleich zehn ist, wobei die Summe aus m4 + m5 + m6 + m7 größer als null und kleiner oder gleich zehn ist;
β) optional bis zu 40 Mol-% einer Verbindung der Formel (III'): 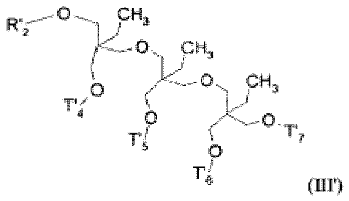 oder von deren Isomer der Formel (IV'): 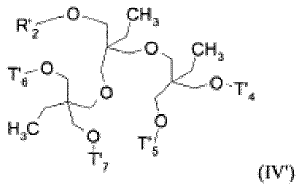 oder von dem Gemisch aus diesen zwei Isomeren, wobei in den Formeln (III') und (IV'):
- R'₂ ein geradkettiges oder verzweigtes Alkylradikal mit 14 Kohlenstoffatomen darstellt,
- T'₄ ein Wasserstoffatom oder ein (-CH₂-CH₂-O-)ₘ₄-H-Radikal darstellt, wobei m4 eine ganze Zahl ist, die größer oder gleich null und kleiner oder gleich zehn ist,
- T'₅, das mit T'₄ identisch oder von ihm verschieden ist, ein Wasserstoffatom oder ein (-CH₂-CH₂-O-)ₘ₅-H-Radikal darstellt, wobei m5 eine ganze Zahl ist, die größer oder gleich null und kleiner oder gleich zehn ist,
- T'₆, das mit T'₄ und T'₅ identisch oder von ihnen verschieden ist, ein Wasserstoffatom oder ein (-CH₂-CH₂-O-)ₘ₆-H-Radikal darstellt, wobei m6 eine ganze Zahl ist, die größer oder gleich null und kleiner oder gleich zehn ist, und
- T'₇, das mit T'₄, T'₅ und T'₆ identisch oder von ihnen verschieden ist, ein Wasserstoffatom oder ein (-CH₂-CH₂-O-)ₘ₇-H-Radikal darstellt, wobei m7 eine ganze Zahl ist, die größer oder gleich null und kleiner oder gleich zehn ist,
- wobei die Summe aus m4 + m5 + m6 + m7 größer als null und kleiner oder gleich zehn ist; und
γ) optional bis zu 10 Mol-% einer Verbindung der Formel (III"): 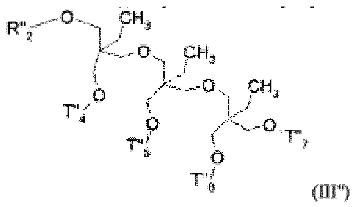 oder von deren Isomer der Formel (IV"): 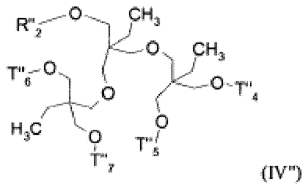 oder von dem Gemisch aus diesen zwei Isomeren, wobei in den Formeln (III") und (IV"):
- R"₂ ein geradkettiges oder verzweigtes Alkylradikal mit 16 Kohlenstoffatomen darstellt,
- T"₄ ein Wasserstoffatom oder ein (-CH₂-CH₂-O-)ₘ₄-H-Radikal darstellt, wobei m4 eine ganze Zahl ist, die größer oder gleich null und kleiner oder gleich zehn ist,
- T"₅, das mit T"₄ identisch oder von ihm verschieden ist, ein Wasserstoffatom oder ein (-CH₂-CH₂-O-)ₘ₅-H-Radikal darstellt, wobei m5 eine ganze Zahl ist, die größer oder gleich null und kleiner oder gleich zehn ist,
- T"₆, das mit T"₄ und T"₅ identisch oder von ihnen verschieden ist, ein Wasserstoffatom oder ein (-CH₂-CH₂-O-)ₘ₆-H-Radikal darstellt, wobei m6 eine ganze Zahl ist, die größer oder gleich null und kleiner oder gleich zehn ist, und
- T"₇, das mit T"₄, T"₅ und T"₆ identisch oder von ihnen verschieden ist, ein Wasserstoffatom oder ein (-CH₂-CH₂-O-)ₘ₇-H-Radikal darstellt, wobei m7 eine ganze Zahl ist, die größer oder gleich null und kleiner oder gleich zehn ist, wobei die Summe aus m4 + m5 + m6 + m7 größer als null und kleiner oder gleich zehn ist;
d) 15 Masse-% bis 45 Masse-% mindestens eines Öls; und
e) 0 Masse-% bis 5 Masse-% Wasser.

2. Zusammensetzung nach Anspruch 1, bei der in der Formel (I) das Radikal R1 ein Laurylradikal oder ein Stearylradikal darstellt.

3. Zusammensetzung nach einem der Ansprüche 1 oder 2, bei der in der Formel (I) n größer oder gleich zwei und kleiner oder gleich zwanzig ist.

4. Zusammensetzung nach einem der Ansprüche 1 bis 3, bei der in dem Polyelektrolyten P die Monomereinheiten mit einer starken Säurefunktion aus 2-Methyl-2-[(1-oxo-2-propenyl)amino]-1-propansulfonsäure hervorgegangen sind, die teilweise oder vollständig in Natriumsalz, in Kaliumsalz oder in Ammoniumsalz überführt ist, und die Monomereinheiten mit einer schwachen Säurefunktion aus Acrylsäure oder Methacrylsäure hervorgegangen sind, die teilweise in Natriumsalz, in Kaliumsalz oder in Ammoniumsalz überführt ist.

5. Zusammensetzung nach einem der Ansprüche 1 bis 4, bei der der Polyelektrolyt P 0,5 Mol-% bis 10 Mol-% einer Monomereinheit enthält, die aus dem Monomer der Formel (I), wie diese oben definiert ist, hervorgegangen ist.

6. Zusammensetzung nach einem der Ansprüche 1 bis 5, bei der die Tensidzusammensetzung (C) ferner Folgendes enthält:
3) bis zu 5 Mol-% einer Zusammensetzung (C_{V}), enthaltend für 100 Mol-%:
α) 60 Mol-% bis 100 Mol-% einer Verbindung der Formel (V): 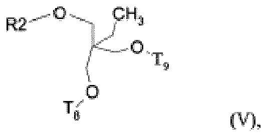 worin:
- R2 ein geradkettiges oder verzweigtes Alkylradikal mit 12 Kohlenstoffatomen darstellt,
- T₈ ein Wasserstoffatom oder ein (-CH₂-CH₂-O-)ₘ₈-H-Radikal darstellt, wobei m8 eine ganze Zahl ist, die größer oder gleich null und kleiner oder gleich zehn ist,
- T₉, das mit T₈ identisch oder von ihm verschieden ist, ein Wasserstoffatom oder ein (-CH₂-CH₂-O-)ₘ₉-H-Radikal darstellt, wobei m9 eine ganze Zahl ist, die größer oder gleich null und kleiner oder gleich zehn ist, und
- wobei die Summe aus m8 + m9 größer als null und kleiner oder gleich zehn ist;
β) optional bis zu 40 Mol-% einer Verbindung der Formel (V'): 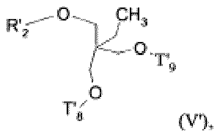 worin:
- R'₂ ein geradkettiges oder verzweigtes Alkylradikal mit 14 Kohlenstoffatomen darstellt,
- T'₈ ein Wasserstoffatom oder ein (-CH₂-CH₂-O-)ₘ₈-H-Radikal darstellt, wobei m8 eine ganze Zahl ist, die größer oder gleich null und kleiner oder gleich zehn ist,
- T'₉, das mit T'₈ identisch oder von ihm verschieden ist, ein Wasserstoffatom oder ein (-CH₂-CH₂-O-)ₘ₉-H-Radikal darstellt, wobei m9 eine ganze Zahl ist, die größer oder gleich null und kleiner oder gleich zehn ist, und
- wobei die Summe aus m8 + m9 größer als null und kleiner oder gleich zehn ist; und
γ) optional bis zu 10 Mol-% einer Verbindung der Formel (V"): 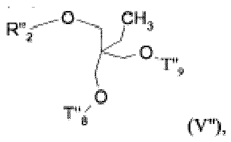 worin:
- R"₂ ein geradkettiges oder verzweigtes Alkylradikal mit 16 Kohlenstoffatomen darstellt,
- T"₉ ein Wasserstoffatom oder ein (-CH₂-CH₂-O-)ₘ₈-H-Radikal darstellt, wobei m8 eine ganze Zahl ist, die größer oder gleich null und kleiner oder gleich zehn ist,
- T"₉, das mit T"₈ identisch oder von ihm verschieden ist, ein Wasserstoffatom oder ein (-CH₂-CH₂-O-)ₘ₉-H-Radikal darstellt, wobei m9 eine ganze Zahl ist, die größer oder gleich null und kleiner oder gleich zehn ist, und
- wobei die Summe aus m8 + m9 größer als null und kleiner oder gleich zehn ist;

7. Zusammensetzung nach einem der Ansprüche 1 bis 6, bei der die Tensidzusammensetzung (C) ferner Folgendes enthält:
4) bis zu 5 Mol-% einer Zusammensetzung (C_{VI}), enthaltend für 100 Mol-%:
α) 60 Mol-% bis 100 Mol-% einer Verbindung der Formel (VI):
R2-(-CH₂-CH₂-O-)ₘ₁₀-H (VI),
worin R2 ein geradkettiges oder verzweigtes aliphatisches Radikal mit 12 Kohlenstoffatomen ist und m10 eine ganze Zahl größer oder gleich null und kleiner oder gleich zehn ist;
β) optional bis zu 40 Mol-% einer Verbindung der Formel (V'):
R'₂-(-CH₂-CH₂-O-)ₘ₁₀-H (VI'),
worin R'₂ ein geradkettiges oder verzweigtes aliphatisches Radikal mit 14 Kohlenstoffatomen ist und m10 eine ganze Zahl größer oder gleich null und kleiner oder gleich zehn ist; und
γ) optional bis zu 10 Mol-% einer Verbindung der Formel (VI"):
R'₂-(-CH₂-CH₂-O-)ₘ₁₀-H (VI"),
worin R"₂ ein geradkettiges oder verzweigtes aliphatisches Radikal mit 16 Kohlenstoffatomen ist und m10 eine ganze Zahl größer oder gleich null und kleiner oder gleich zehn ist.

8. Zusammensetzung nach einem der Ansprüche 1 bis 7, bei der die Tensidzusammensetzung (C) Folgendes enthält:
1) einen Anteil von größer oder gleich 20 Mol-% und kleiner oder gleich 50 Mol-% einer Zusammensetzung (C_{II}), wie diese oben definiert ist;
2) einen Anteil von größer oder gleich 50 Mol-% und kleiner oder gleich 80 Mol-% einer Zusammensetzung (C_{III}), wie diese oben definiert ist.

9. Zusammensetzung nach einem der Ansprüche 1 bis 8, bei der in der Tensidzusammensetzung (C)
1) die Zusammensetzung (C_{II}) für 100 Mol-% Folgendes enthält:
α) 60 Mol-% bis 80 Mol-% der Verbindung der Formel (II),
ß) 15 Mol-% bis 30 Mol-% der Verbindung der Formel (II'), und
γ) bis zu 10 Mol-% der Verbindung der Formel (II"), und
2) die Zusammensetzung (C_{III}) für 100 Mol-% Folgendes enthält:
α) 60 Mol-% bis 80 Mol-% der Verbindung der Formel (III) oder von deren Isomer der Formel (IV) oder von dem Gemisch aus diesen Isomeren,
β) 15 Mol-% bis 30 Mol-% der Verbindung der Formel (III') oder von deren Isomer der Formel (IV') oder von dem Gemisch aus diesen Isomeren und
γ) bis zu 10 Mol-% der Verbindung der Formel (III") oder von deren Isomer der Formel (IV") oder von dem Gemisch aus diesen Isomeren.

10. Zusammensetzung nach einem der Ansprüche 1 bis 9, bei der das Emulgatorsystem (S₂) des Typs Öl-in-Wasser (O/W) aus 100 Masse-% der Tensidzusammensetzung (C) besteht.

11. Zusammensetzung nach einem der Ansprüche 1 bis 10, bei der das Emulgatorsystem (S₂) des Typs Öl-in-Wasser (O/W) für 100 % seiner Masse Folgendes enthält:
- 10 Masse-% bis 40 Masse-% heptaethoxylierten Laurylalkohols und
- 60 Masse-% bis 90 Masse-% der Tensidzusammensetzung (C).

12. Zusammensetzung nach einem der Ansprüche 1 bis 11, bei der in dem Polyelektrolyten P die aus dem Monomer der Formel (I) hervorgegangene Monomereinheit eine Monomereinheit ist, die aus tetraethoxyliertem Laurylacrylat hervorgegangen ist.

13. Zusammensetzung nach Anspruch 12, in der der Polymerelektrolyt (P) ausgewählt ist aus:
- den vernetzten Copolymeren von Acrylsäure, die teilweise in ein Salz überführt ist in Form von Natrium- oder Ammoniumsalz, von Acrylamid und von tetraethoxyliertem Laurylacrylat;
- den vernetzten Copolymeren von 2-Methyl-2-[(1-oxo-2-propenyl)amino]-1-propansulfonsäure, die teilweise in ein Salz überführt ist in Form von Natriumsalz oder von Ammoniumsalz, von Acrylamid und von tetraethoxyliertem Laurylacrylat;
- den vernetzten Copolymeren von 2-Methyl-2-[(1-oxo-2-propenyl)amino]-1-propansulfonsäure, die teilweise in ein Salz überführt ist in Form von Natriumsalz oder von Ammoniumsalz, von 2-Hydroxyethylacrylat und von tetraethoxyliertem Laurylacrylat;
- den vernetzten Copolymeren von Acrylamid, von 2-Methyl-2-[(1-oxo-2-propenyl)amino]-1-propansulfonsäure, von Acrylsäure, die teilweise in ein Salz überführt ist in Form von Natriumsalz oder von Ammoniumsalz, und von tetraethoxyliertem Laurylacrylat;
- den Copolymeren von 2-Methyl-2-[(1-oxo-2-propenyl)amino]-1-propansulfonsäure, die teilweise in ein Salz überführt ist in Form von Natriumsalz oder von Ammoniumsalz, von Acrylamid, von Vinylpyrrolidon und von tetraethoxyliertem Laurylacrylat; und
- den vernetzten Copolymeren von 2-Methyl-2-[(1-oxo-2-propenyl)amino]-1-propansulfonsäure, die teilweise oder vollständig in ein Salz überführt ist in Form von Natriumsalz, von Acrylsäure, die teilweise in ein Salz überführt ist in Form von Natriumsalz oder von Ammoniumsalz, von 2-Hydroxyethylacrylat, von Tris(hydroxymethyl)aminomethylacrylamid und von tetraethoxyliertem Laurylacrylat.

14. Zusammensetzung nach einem der Ansprüche 1 bis 13, bei der der vernetzte anionische Polymerelektrolyt (P) für 100 % verwendeter Monomere Folgendes aufweist:
- 40 Mol-% bis 80 Mol-% Monomereinheiten, die aus einem Monomer hervorgegangen sind, das eine starke Säurefunktion aufweist;
- 15 Mol-% bis 55 Mol-% Monomereinheiten, die aus einem neutralen Monomer hervorgegangen sind, der sich von der Verbindung der Formel (I), wie diese oben definiert ist, unterscheidet;
- 1 Mol-% bis 5 Mol-% Monomereinheiten, die aus einem Monomer der Formel (I) hervorgegangen sind, wie diese oben definiert ist.

15. Verwendung der Zusammensetzung nach einem der Ansprüche 1 bis 14 als Verdickungsmittel und/oder Emulgator zur Herstellung einer topischen kosmetischen, dermopharmazeutischen oder pharmazeutischen Zusammensetzung.

16. Topische kosmetische, dermopharmazeutische oder pharmazeutische Zusammensetzung, **dadurch gekennzeichnet, dass** sie als Verdickungsmittel und/oder Emulgator eine wirksame Menge der Zusammensetzung nach einem der Ansprüche 1 bis 14 enthält.

## Claims

1. Composition in the form of a self-invertible inverse latex comprising, for 100 % of its weight:
a) - from 50 wt.% to 70 wt.% of a cross-linked anionic polyelectrolyte (P) obtained by polymerisation:
- of at least one neutral monomer of formula (I): 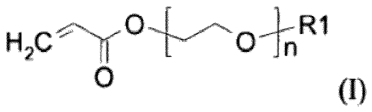 in which the radical R1 represents a linear or branched aliphatic radical comprising from 8 to 20 carbon atoms and n represents a number greater than or equal to one and less than or equal to thirty;
- of at least one neutral monomer selected from acrylamide, N,N-dimethylacrylamide, N-[2-hydroxy-1,1-bis(hydroxymethyl)ethyl]propenamide [or tris(hydroxy-methyl)acrylamidomethane or N-[tris(hydroxymethyl)-methyl]acrylamide, also known as THAM] or (2-hydroxyethyl) acrylate; and
- of at least one monomer comprising a strong acid function and/or of at least one monomer comprising a weak acid function;
b) - from 4 wt.% to 10 wt.% of an emulsifying system (S₁) of water-in-oil (W/O) type;
**c)** - from 1 wt.% to 10 wt.% of an emulsifying system (S₂) of oil-in-water (O/W) type comprising a non-zero proportion by weight of a surfactant composition (C), said surfactant composition (C) comprising, for 100 mol.%:
1) - a proportion of greater than or equal to 10 mol.% and of less than or equal to 50 mol.% of a composition (C_{II}) comprising, per 100 mol.%:
**α)** - from 60 mol.% to 100 mol.% of a compound of formula (II): 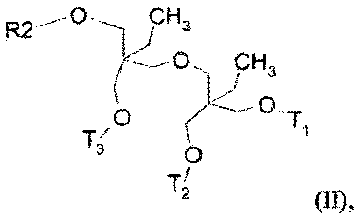 in which:
- R2 represents a linear or branched alkyl radical comprising 12 carbon atoms,
- T₁ represents a hydrogen atom or a (-CH₂-CH₂-O-)ₘ₁-H radical in which m1 is an integer greater than or equal to zero and less than or equal to ten,
- T₂, which is identical to or different from T₁, represents a hydrogen atom or a (-CH₂-CH₂-O-)ₘ₂-H radical in which m2 is an integer greater than or equal to zero and less than or equal to ten, and
- T₃, which is identical to or different from T₁ and T₂, represents a hydrogen atom or a (-CH₂-CH₂-O-)ₘ₃-H radical in which m3 is an integer greater than or equal to zero and less than or equal to ten,
- it being understood that the sum m1+m2+m3 is greater than 0 and less than or equal to ten;
**β)** - optionally up to 40 mol.% of a compound of formula (II'): 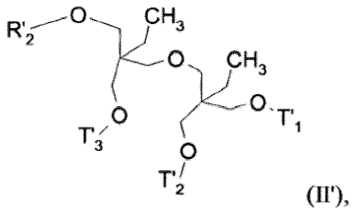 in which:
- R'₂ represents a linear or branched alkyl radical comprising 14 carbon atoms,
- T'₁ represents a hydrogen atom or a (-CH₂-CH₂-O-)ₘ₁-H radical in which m1 is an integer greater than or equal to zero and less than or equal to ten,
- T'₂, which is identical to or different from T'₁, represents a hydrogen atom or a (-CH₂-CH₂-O-)ₘ₂-H radical in which m2 is an integer greater than or equal to zero and less than or equal to ten, and
- T'₃, which is identical to or different from T'₁ and T'₂, represents a hydrogen atom or a (-CH₂-CH₂-O-)ₘ₃-H radical in which m3 is an integer greater than or equal to zero and less than or equal to ten,
- it being understood that the sum m1 +m2+m3 is greater than 0 and less than or equal to ten; and
**γ)** - optionally up to 10 mol.% of a compound of formula (II"): 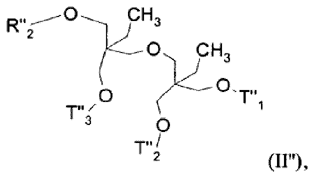 in which:
- R"₂ represents a linear or branched alkyl radical comprising 16 carbon atoms,
- T"₁ represents a hydrogen atom or a (-CH₂-CH₂-O-)ₘ₁-H radical in which m1 is an integer greater than or equal to zero and less than or equal to ten,
- T"₂, which is identical to or different from T"₁, represents a hydrogen atom or a (-CH₂-CH₂-O-)ₘ₂-H radical in which m2 is an integer greater than or equal to zero and less than or equal to ten, and
- T"₃, which is identical to or different from T"₁ and T"₂, represents a hydrogen atom or a (-CH₂-CH₂-O-)ₘ₃-H radical in which m3 is an integer greater than or equal to zero and less than or equal to ten,
- it being understood that the sum m1+m2+m3 is greater than 0 and less than or equal to ten;
2) - a proportion of greater than or equal to 50 mol.% and of less than or equal to 90 mol.% of a composition (C_{III}) comprising, per 100 mol.%:
**α)** - from 60 mol.% to 100 mol.% of a compound of formula (III): 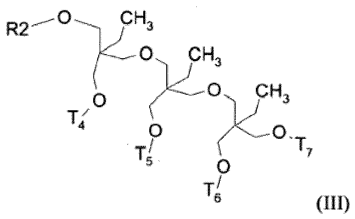 or of its isomer of formula (IV): 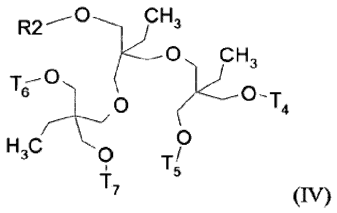 or of the mixture of these two isomers,
in which formulae (III) and (IV):
- R₂ represents a linear or branched alkyl radical comprising 12 carbon atoms,
- T₄ represents a hydrogen atom or a (-CH₂-CH₂-O-)ₘ₄-H radical in which m4 is an integer greater than or equal to zero and less than or equal to ten,
- T₅, which is identical to or different from T₄, represents a hydrogen atom or a (-CH₂-CH₂-O-)ₘ₅-H radical in which m5 is an integer greater than or equal to zero and less than or equal to ten,
- T₆, which is identical to or different from T₄ and T₅, represents a hydrogen atom or a (-CH₂-CH₂-O-)ₘ₆-H radical in which m6 is an integer greater than or equal to zero and less than or equal to ten,
- T₇, which is identical to or different from T₄, T₅ and T₆, represents a hydrogen atom or a (-CH₂-CH₂-O-)ₘ₇-H radical in which m7 is an integer greater than or equal to zero and less than or equal to ten, it being understood that the sum m4+m5+m6+m7 is greater than 0 and less than or equal to ten;
**β)** - optionally up to 40 mol.% of a compound of formula (III'): 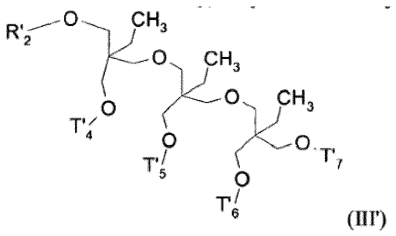 or of its isomer of formula (IV'): 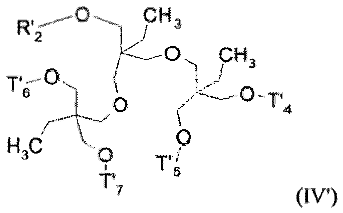 or of the mixture of these two isomers,
in which formulae (III') and (IV'):
- R'₂ represents a linear or branched alkyl radical comprising 14 carbon atoms,
- T'₄ represents a hydrogen atom or a (-CH₂-CH₂-O-)ₘ₄-H radical in which m4 is an integer greater than or equal to zero and less than or equal to ten,
- T'₅, which is identical to or different from T'₄, represents a hydrogen atom or a (-CH₂-CH₂-O-)ₘ₅-H radical in which m5 is an integer greater than or equal to zero and less than or equal to ten,
- T'₆, which is identical to or different from T'₄ and T'₅, represents a hydrogen atom or a (-CH₂-CH₂-O-)ₘ₆-H radical in which m6 is an integer greater than or equal to zero and less than or equal to ten, and
- T'₇, which is identical to or different from T'₄, T'₅ and T'₆, represents a hydrogen atom or a (-CH₂-CH₂-O-)ₘ₇-H radical in which m7 is an integer greater than or equal to zero and less than or equal to ten,
- it being understood that the sum m4+m5+m6+m7 is greater than 0 and less than or equal to ten; and
**γ)** - optionally up to 10 mol.% of a compound of formula (III"): 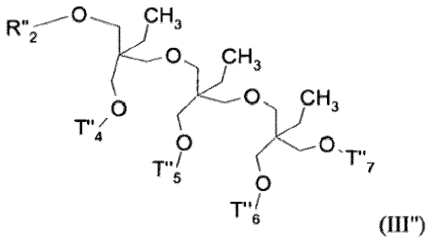 or of its isomer of formula (IV"): 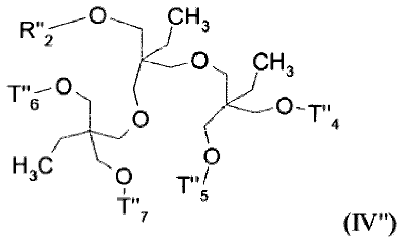 or of the mixture of these two isomers,
in which formulae (III") and (IV"):
- R"₂ represents a linear or branched alkyl radical comprising 16 carbon atoms,
- T"₄ represents a hydrogen atom or a (-CH₂-CH₂-O-)ₘ₄-H radical in which m4 is an integer greater than or equal to zero and less than or equal to ten,
- T"₅, which is identical to or different from T"₄, represents a hydrogen atom or a (-CH₂-CH₂-O-)ₘ₅-H radical in which m5 is an integer greater than or equal to zero and less than or equal to ten,
- T"₆, which is identical to or different from T"₄ and T"₅, represents a hydrogen atom or a (-CH₂-CH₂-O-)ₘ₆-H radical in which m6 is an integer greater than or equal to zero and less than or equal to ten, and
- T"₇, which is identical to or different from T"₄, T"₅ and T"₆, represents a hydrogen atom or a (-CH₂-CH₂-O-)ₘ₇-H radical in which m7 is an integer greater than or equal to zero and less than or equal to ten,
- it being understood that the sum m4+m5+m6+m7 is greater than 0 and less than or equal to ten;
**d) -** from 15 wt.% to 45 wt.% of at least one oil, and
**e) -** from 0 wt.% to 5 wt.% of water.

2. Composition according to claim 1, wherein, in formula (I), the radical R1 represents a lauryl radical or a stearyl radical.

3. Composition according to either claim 1 or claim 2, wherein, in formula (I), n is greater than or equal to two and less than or equal to twenty.

4. Composition according to any of claims 1 to 3, wherein, in the polyelectrolyte P, the monomer units comprising a strong acid function result from 2-methyl-2-[(1-oxo-2-propenyl)amino]-1-propanesulfonic acid partially or completely salified as sodium salt, as potassium salt or as ammonium salt and the monomer units comprising a weak acid function result from acrylic acid or from methacrylic acid partially salified as sodium salt, as potassium salt or as ammonium salt.

5. Composition according to any of claims 1 to 4, wherein the polyelectrolyte P comprises, in mole percent, from 0.5 % to 10 % of a monomer unit resulting from the monomer of formula (I) as defined above.

6. Composition according to any of claims 1 to 5, wherein said surfactant composition (C) additionally comprises:
**3) -** up to 5 mol.% of a composition (C_{V}) comprising, per 100 mol.%:
**α)** - from 60 mol.% to 100 mol.% of a compound of formula (V): 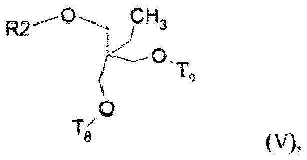 in which:
- R2 represents a linear or branched alkyl radical comprising 12 carbon atoms,
- T₈ represents a hydrogen atom or a (-CH₂-CH₂-O-)ₘ₈-H radical in which m8 is an integer greater than or equal to zero and less than or equal to ten,
- T₉, which is identical to or different from T₈, represents a hydrogen atom or a (-CH₂-CH₂-O-)ₘ₉-H radical in which m9 is an integer greater than or equal to zero and less than or equal to ten, and
- it being understood that the sum m8+m9 is greater than 0 and less than or equal to ten;
**β)** - optionally up to 40 mol.% of a compound of formula (V'): 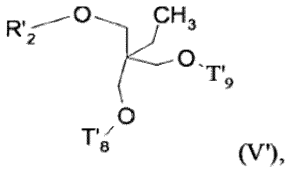 in which:
- R'₂ represents a linear or branched alkyl radical comprising 14 carbon atoms,
- T'₈ represents a hydrogen atom or a (-CH₂-CH₂-O-)ₘ₈-H radical in which m8 is an integer greater than or equal to zero and less than or equal to ten,
- T'₉, which is identical to or different from T'₈, represents a hydrogen atom or a (-CH₂-CH₂-O-)ₘ₉-H radical in which m9 is an integer greater than or equal to zero and less than or equal to ten, and
- it being understood that the sum m8+m9 is greater than 0 and less than or equal to ten; and
**γ)** - optionally up to 10 mol.% of a compound of formula (V"): 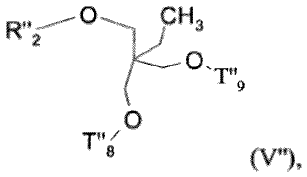 in which:
- R"₂ represents a linear or branched alkyl radical comprising 16 carbon atoms,
- T"₈ represents a hydrogen atom or a (-CH₂-CH₂-O-)ₘ₈-H radical in which m8 is an integer greater than or equal to zero and less than or equal to ten,
- T"₉, which is identical to or different from T"₈, represents a hydrogen atom or a (-CH₂-CH₂-O-)ₘ₉-H radical in which m9 is an integer greater than or equal to zero and less than or equal to ten, and
- it being understood that the sum m8+m9 is greater than 0 and less than or equal to ten.

7. Composition according to any of claims 1 to 6, wherein said surfactant composition (C) additionally comprises:
**4) -** up to 5 mol.% of a composition (C_{VI}) comprising, per 100 mol.%:
**α)** - from 60 mol.% to 100 mol.% of a compound of formula (VI):
R2-(-CH₂-CH₂-O-)ₘ₁₀-H (VI),
in which R2 represents a linear or branched alkyl radical comprising 12 carbon atoms and m10 is an integer greater than or equal to zero and less than or equal to ten;
**β)** - optionally up to 40 mol.% of a compound of formula (VI'):
R'₂-(-CH₂-CH₂-O-)ₘ₁₀-H (VI'),
in which R'₂ represents a linear or branched alkyl radical comprising 14 carbon atoms and m10 is an integer greater than or equal to zero and less than or equal to ten; and
**γ)** - optionally up to 10 mol.% of a compound of formula (VI"):
R"₂-(-CH₂-CH₂-O-)ₘ₁₀-H (VI"),
in which R"₂ represents a linear or branched alkyl radical comprising 16 carbon atoms and m10 is an integer greater than or equal to zero and less than or equal to ten.

8. Composition according to any of claims 1 to 7, wherein said surfactant composition (C) comprises:
1) - a proportion of greater than or equal to 20 mol.% and of less than or equal to 50 mol.% of a composition (C_{II}) as defined above;
2) - a proportion of greater than or equal to 50 mol.% and of less than or equal to 80 mol.% of a composition (C_{III}) as defined above.

9. Composition according to any of claims 1 to 8, wherein, in said surfactant composition (C):
1) - said composition (C_{II}) comprises, per 100 mol.%:
**α)** - from 60 mol.% to 80 mol.% of the compound of formula (II),
**β)** - from 15 mol.% to 30 mol.% of the compound of formula (II'), and
**γ)** - up to 10 mol.% of the compound of formula (II"), and
2) - said composition (C_{III}) comprises, per 100 mol.%:
**α)** - from 60 mol.% to 80 mol.% of the compound of formula (III), of its isomer of formula (IV) or of the mixture of these isomers,
**β)** - from 15 mol.% to 30 mol.% of the compound of formula (III'), of its isomer of formula (IV') or of the mixture of these isomers, and
**γ)** - up to 10 mol.% of the compound of formula (III"), of its isomer of formula (IV") or of the mixture of these isomers.

10. Composition according to any of claims 1 to 9, wherein said emulsifying system (S₂) of oil-in-water (O/W) type consists of 100 wt.% of said surfactant composition (C).

11. Composition according to any of claims 1 to 10, wherein said emulsifying system (S₂) of oil-in-water (O/W) type comprises, per 100 % of its weight:
- from 10 wt.% to 40 wt.% of heptaethoxylated lauryl alcohol and
- from 60 wt.% to 90 wt.% of said surfactant composition (C).

12. Composition according to any of claims 1 to 11, wherein, in the polyelectrolyte (P), the monomer unit resulting from the monomer of formula (I) is a monomer unit resulting from tetraethoxylated lauryl acrylate.

13. Composition according to claim 12, wherein the polyelectrolyte (P) is selected from:
- cross-linked copolymers of acrylic acid partially salified in the sodium salt or ammonium salt form, of acrylamide and of tetraethoxylated lauryl acrylate;
- cross-linked copolymers of 2-methyl-2-[(1-oxo-2-propenyl)amino]-1-propanesulfonic acid partially salified in the sodium salt or ammonium salt form, of acrylamide and of tetraethoxylated lauryl acrylate;
- cross-linked copolymers of 2-methyl-2-[(1-oxo-2-propenyl)amino]-1-propanesulfonic acid partially salified in the sodium salt or ammonium salt form, of 2-hydroxyethyl acrylate and of tetraethoxylated lauryl acrylate;
- cross-linked copolymers of acrylamide, of 2-methyl-2-[(1-oxo-2-propenyl)amino]-1-propanesulfonic acid, of acrylic acid partially salified in the sodium salt or ammonium salt form and of tetraethoxylated lauryl acrylate;
- copolymers of 2-methyl-2-[(1-oxo-2-propenyl)amino]-1-propanesulfonic acid partially salified in the sodium salt or ammonium salt form, of acrylamide, of vinylpyrrolidone and of tetraethoxylated lauryl acrylate; and
- cross-linked copolymers of 2-methyl-2-[(1-oxo-2-propenyl)amino]-1-propanesulfonic acid partially or completely salified in the sodium salt form, of acrylic acid partially salified in the sodium salt or ammonium salt form, of 2-hydroxyethyl acrylate, of tris(hydroxymethyl)aminomethyl acrylamide and of tetraethoxylated lauryl acrylate.

14. Composition according to any of claims 1 to 13, wherein the cross-linked anionic polyelectrolyte (P) comprises, per 100 % of monomers used:
- from 40 mol.% to 80 mol.% of monomer units resulting from a monomer comprising a strong acid function;
- from 15 mol.% to 55 mol.% of monomer units resulting from a neutral monomer other than the compound of formula (I) as defined above;
- from 1 mol.% to 5 mol.% of monomer units resulting from a monomer of formula (I) as defined above.

15. Use of the composition according to any of claims 1 to 14 as a thickening and/or emulsifying agent for preparing a cosmetic, dermopharmaceutical or pharmaceutical topical composition.

16. Cosmetic, dermopharmaceutical or pharmaceutical topical composition, **characterised in that** it comprises, as a thickening and/or emulsifying agent, an effective amount of the composition according to any of claims 1 to 14.
